(19) Europäisches Patentamt
European Patent Office
Office européen des brevets

(11) EP 3 090 738 A1

(12) **EUROPEAN PATENT APPLICATION**

(43) Date of publication:
**09.11.2016 Bulletin 2016/45**

(21) Application number: **15382229.1**

(22) Date of filing: **04.05.2015**

(51) Int Cl.:
***A61K 31/19*** (2006.01) ***A61K 31/525*** (2006.01)
***A61P 9/10*** (2006.01) ***A61P 25/00*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicants:
- **Universidade de Santiago de Compostela**
  **15782 Santiago de Compostela (ES)**
- **Servizo Galego de Saúde (SERGAS)**
  **15006 Santiago de Compostela (ES)**
- **Fundación Ramón Domínguez**
  **15706 Santiago de Compostela (ES)**

(72) Inventors:
- **Brea Floriani, José Manuel**
  **15782 Santiago de Compostela (ES)**
- **Campos Pérez, Francisco**
  **15706 Santiago de Compostela (ES)**
- **Castillo Sánchez, José**
  **15006 Santiago de Compostela (ES)**
- **Loza García, Mª Isabel**
  **15782 Santiago de Compostela (ES)**
- **Sobrino Moreiras, Tomás**
  **15006 Santiago de Compostela (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

# (54) RIBOFLAVIN FOR THE TREATMENT OF ISCHEMIC STROKE AND/OR OTHER GLUTAMATE EXCITOTOXICITY-ASSOCIATED DISEASES

(57) The present invention relates to a combination therapy comprising the use of riboflavin and oxaloacetate. Specifically, it is directed to new compositions, pharmaceutical compositions, kits of parts and methods for treating ischemic stroke and/or other excitotoxicity-associated diseases. It further relates to riboflavin for use in the therapeutic treatment of acute ischemic stroke by intravascular administration within the first twelve hours of symptom onset.

EP 3 090 738 A1

## Description

### FIELD OF THE INVENTION

The present invention relates to the pharmaceutical field, in particular to the field of new drug development for the treatment of glutamate excitotoxicity-associated diseases. The present invention relates to a combination therapy comprising the use of riboflavin and oxaloacetate. Specifically, it is directed to new compositions, pharmaceutical compositions, kits of parts and methods for treating ischemic stroke and/or other excitotoxicity-associated diseases. It further relates to riboflavin for use in the therapeutic treatment of acute ischemic stroke by intravascular administration within the first twelve hours of symptom onset.

### BACKGROUND OF THE INVENTION

Cerebral ischemia, also known as ischemic stroke or stroke, is a leading cause of mortality and morbidity in developed countries, with increasing incidence because of the progressive aging of the population. Pharmacological or mechanical reperfusion therapy is the unique effective treatment during the acute phase of ischemic stroke and it is associated with good outcome in 50-70% of cases. However, these treatments are only applicable to <10% of patients because of risks related to hemorrhage with a short therapeutic window, not longer than 4.5 hours after the onset of symptoms. Owing to these therapeutic limitations, the development of new and effective therapies to be used during the acute phase of ischemic stroke is in high demand (Tomsick TA et al., Neurology, 2010, 74: 1069-1076).

After ischemic stroke, there is a rapid but transient elevation of glutamate into the extracellular space followed by a marked increase in intracellular calcium, which provokes a neuronal death through an excitotoxicity mechanism. Consequently, calcium and glutamate antagonists have been widely studied as protective agents in experimental models of cerebral ischemia with encouraging results. Unfortunately, they failed or displayed severe adverse effects when they were tested in clinical trials, as hallucinations, agitations, catatonia, peripheral sensory loss, nausea, and elevation in blood pressure (Jia M et al., CNS Drugs. 2015; 29:153-162). Nevertheless, because of the central role of glutamate in the ischemic cascade, the mitigation of glutamate excitotoxicity remains one of the most promising strategies for the development of effective treatments to minimize neurological damage following acute ischemic stroke. In this sense, novel therapeutic approaches aim at reducing the increased glutamate levels produced early after the acute phase of ischemia (see, Lipton P. et al., Physiol Rev., 1999, 79:1431-1568 and Ginsberg MD. et al., Neuropharmacology, 2008, 55:363-389).

Previous studies from our laboratory have shown that a decrease in blood glutamate concentration leads to a larger natural glutamate gradient between the brain and blood, facilitating the efflux of excess extracellular brain glutamate into the blood. Reduction of glutamate concentration in blood is induced through the activity of the blood-resident enzyme glutamate oxaloacetate transaminase (GOT), which catalyzes the reversible transformation of oxaloacetate and glutamate to aspartate and $\alpha$-ketoglutarate, as shown below. Thus, the increase in the co-substrate (oxaloacetate) shifts the equilibrium of the reaction to the right side, thereby decreasing blood glutamate concentration and lowering the brain glutamate levels (see, Teichberg et al., Neuroscience, 2009, 158:301-308 and Campos et al., Int J Biochem Cell Biol. 2012;44:262-265).

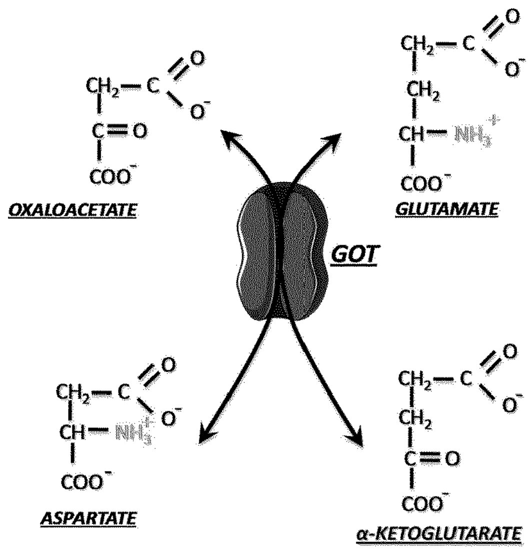

Based on this mechanism, the inventors previously demonstrated that the intravenous (i.v.) administration of oxaloacetate in a rat model of ischemia induced by transient occlusion of the middle cerebral artery occlusion (MCAO) decreases the glutamate concentration in blood and induces a lowering of infarct volume and edema after ischemia. These effects were also associated with a significant reduction in the sensorimotor deficit. To confirm that the protective effect was mediated by a decrease in the brain glutamate levels, magnetic resonance spectroscopy (MRS) was performed in the infarct region. Spectroscopic analysis revealed that the increase in brain glutamate seen in control animals after MCAO was significantly reduced in animals treated with oxaloacetate. These experimental results showed that systemic reduction in glutamate concentration induced by means of exogenous oxaloacetate administration could be used as a novel and efficient protective strategy in ischemic stroke (see, Campos F et al., J Cereb Blood Flow Metab., 2011, 31:1378-1386 and WO2011/148014).

Translating the use of oxaloacetate to patients presents however, some important limitations as the required effective dosage, namely that corresponding to the one shown to be effective in rats, might be toxic in humans (Leibowitz A., et al., Int J Mol Sei., 2012, 13:10041-10066, Armstrong C and Staples J. J Comp Physiol B., 2010,180:775-783.).

Therefore, there is a need to find a novel non-toxic drug with efficient glutamate scavenging activity for use in human subjects against excitotoxicity related diseases and in particular against ischemic stroke.

## BRIEF DESCRIPTION OF THE INVENTION

The enzyme glutamate oxaloacetate transaminase (GOT) is a liver enzyme which, in the presence of oxaloacetate, metabolizes glutamate to form aspartate and $\alpha$-ketoglutarate as degradation products as shown in the scheme provided above (see also, Kirsch et al., J Mol Biol 1984, 174:497-525).

It was previously disclosed by the inventors that a therapeutically effective amount of oxaloacetate or its derivatives increases glutamate metabolism, thus decreasing the glutamate concentration in blood. This resulted in a more pronounced glutamate concentration gradient between the brain and the blood, thereby promoting the outflow of extracellular glutamate from the cerebral parenchyma.

The rapid outflow of glutamate released by cells that have lost their energy capabilities, or dead cells, from the cerebral parenchyma results in less damage to healthy tissue and therefore a reduction in the neurological deficit that occurs in those patients with disorders associated with high glutamate concentrations in brain tissue.

The enzyme succinate dehydrogenase (SDH), also known as respiratory Complex II, is an enzyme complex bound to the inner mitochondrial membrane of mammalian mitochondria that participates in both the citric acid cycle and the electron transport chain, and inhibition of this enzyme can cause fatal effects to the cells (see, Rustin P et al., Biochem J. 1991, 274 (Pt 1):249-255.

By preparing dose-response curves of oxaloacetate referred to the activity of the enzyme succinate dehydrogenase (SDH), the inventors have observed that oxaloacetate at high doses induces an inhibition of SDH, see Figure 1. Furthermore, toxic effects of oxaloacetate linked to SDH inhibition have been previously disclosed by other authors (Leibowitz A., et al., Int J Mol Sei., 2012, 13:10041-10066, Armstrong C and Staples J. J Comp Physiol B., 2010, 180:775-783).

The inventors have now found that the combination of riboflavin with low doses of oxaloacetate synergistically modulates GOT activity *in vitro* reducing the formation of glutamate and *in vivo* decreasing glutamate levels in blood, ultimately providing a neuroprotective effect in the absence of the toxic effects linked to SDH inhibition previously disclosed for oxaloacetate.

Thus, in accordance with the particular findings of the present invention, there is provided:

A first aspect of the present invention relates to riboflavin for use in the treatment of a disease associated with glutamate excitotoxicity in a subject in combination with oxaloacetate, wherein said treatment is a prophylactic treatment before the clinical onset of the disease or a therapeutic treatment after the clinical onset of the disease; and wherein combination is herein understood as the simultaneous or sequential administration of oxaloacetate and riboflavin to said subject.

In a second aspect the present invention refers to a composition comprising riboflavin and oxaloacetate.

A third aspect of the invention refers to a kit of parts comprising riboflavin and oxaloacetate, together with instructions for use in the treatment of a disease involving the simultaneous or sequential administration of both active ingredients.

A forth aspect of the present invention relates to riboflavin for use in the therapeutic treatment of acute ischemic stroke by intravascular administration to a subject suffering or suspected to suffer from acute ischemic stroke within the first twelve hours of symptom onset.

In a fifth aspect, the present invention further provides a pharmaceutical composition comprising riboflavin, and a pharmaceutically acceptable carrier, additive or excipient for use according to the fifth aspect of the invention.

## BRIEF DESCRIPTION OF THE FIGURES

**Figure 1: Fig.1A** and **Fig.1B** provides oxaloacetate dose-response curves on GOT and SDH activities, respectively; and **Fig.1C** and **Fig.1D** provides CBG000592 (riboflavin) dose-response curves on GOT and SDH activities, respectively. Duplicate measurements of a representative experiment are shown.

**Figure 2:** Synergic effect on inhibition of glutamate formation of combined administration of CGB000592 (riboflavin) and oxaloacetate, referred to individually administered compounds.

**Figure 3:** Dose-response curves of oxaloacetate and CBG000592 (riboflavin) referred to GOT and SDH activities as those provide in Figure 1, wherein those doses of oxaloacetate and riboflavin, respectively, which have shown to be effective in the inhibition of glutamate formation when used in combination (Figure 2) are indicated with an arrow.

**Figure 4:** Dose-response effect of CBG000592 (riboflavin) 1, 10 and 50 mg/Kg on blood glutamate *in vivo* in healthy animals. Animals treated with saline were used as control group, and animals treated with oxaloacetate 35 mg/Kg as positive control group. Treatments were administered intravenously. Glutamate data are shown as mean±s.e.m. of percentage with respect to basal levels (considered as 100%). N=6 animals per group.

**Figure 5:** Time course of blood glutamate concentration (μM) in MCAO rats. MCAO rats were treated with saline (control group) (n=10), CBG000592 (riboflavin) 1 mg/Kg (n=10), oxaloacetate 35 mg/Kg (n=10); and CBG000592 (riboflavin) 1 mg/Kg in combination with of oxaloacetate (15 mg/Kg). A solid arrow indicates the moment of treatment injection, administered at the moment of reperfusion (45 min after occlusion). Serum glutamate concentration was measured under basal conditions (before surgery), and 1h, 3h and 24h after reperfusion. Data are shown as mean±S.E.M. *P<0.05, **P<0.01 compared with basal levels.

**Figure 6:** In vivo model of ischemia/reperfusion. **Fig.6A** shows the infarct size assessed by means of magnetic resonance imaging (MRI) in MCAO rats. **Fig.6B** and **Fig.6C** show the infarct lesion volume (mm3) and lesion volume relative to basal (%) in MCAO rats after treatment. MCAO rats were treated with saline (control group) (n=10), CBG000592 1 mg/Kg (n=10), oxaloacetate 35 mg/Kg (n=10) and CBG000592 1 mg/Kg in combination with of oxaloacetate (15 mg/Kg). Treatments were administered at the moment of the reperfusion. Infarct sizes were measured over cerebral artery occlusion, at 24 h and 7 days after ischemia. Data are shown as the mean of infarct volume (mm3)±S.E.M **(Fig.6B),** and percentage relative to the basal volume **(Fig.6C**). *P<0.05, **P<0.01 with respect to the control group.

Figure 7: Effect of treatments on somatosensory test. Sensorimotor deficits after an ischemic insult were assessed using the cylinder test and Bederson test. Somatosensory tests were performed 1 day before surgery (baseline), 24 h and day 7 after MCAO. Values represent the mean±S.E.M, *P<0.05, **P<0.01 relative to basal levels.

## DETAILED DESCRIPTION OF THE INVENTION

### DEFINITIONS

The term "disease" as used herein is intended to be generally synonymous, and is used interchangeably with, the terms "pathology", "disorder" and "condition" (as in medical condition), in that all reflect an abnormal condition of the body or of one of its parts that impairs normal functioning and is typically manifested by distinguishing signs and symptoms.

The term "therapeutic treatment" as used herein refers to bringing a body from a pathological state or disease back to its normal, healthy state. For instance, wherein said disease is acute ischemic stroke, after the onset or clinical manifestation of the ischemia, including those patients with clinical suspicion of ischemic stroke (i.e., definitive determination of acute ischemic stroke, typically by neuroimaging, not performed). It is noted that, this term as used herein is not understood to include the term "prophylactic treatment" as defined herein.

The term "prophylactic treatment" as used herein refers to preventing a pathological state. For instance, wherein said disease is acute ischemic stroke, before the onset or clinical manifestation of the disease. It is noted that this term as used herein is not understood to include the term "therapeutic treatment" as defined above.

The term "subject" as used herein refers to a mammalian subject. Preferably, it is selected from a human, companion animal, non-domestic livestock or zoo animal. For example, the subject may be selected from a human, dog, cat, cow, pig, sheep, horse, bear, and so on. In a preferred embodiment, said mammalian subject is a human subject.

The term "suspected to suffer from a disease" as used herein refers to those patients who present clinical symptoms which indicate that said patient is suffering from a disease but the required tests for confirming the diagnosis have not been carried out. For instance, a patient suspected to suffer from acute ischemic stroke.

The term "ischemia symptom onset" as used herein refers to the time point where the subject presents symptoms characteristic of ischemia such as face drooping, arm weakness, headache, trouble with seeing in one or both eyes and speech difficulty.

The term "therapeutically effective amount" as used herein refers to an amount that is effective, upon single or multiple dose administration to a subject (such as a human patient) in the prophylactic or therapeutic treatment of a given disease, preferably in a glutamate excitotoxicity-associated disease.

The term "NIHSS scale" as used herein is a commonly used scale to measure the level of impairment caused by a stroke (Kasner SE. Lancet Neurol. 2006;7:603-12).

The term "pre-stroke modified Rankin scale" is a commonly used scale for measuring the degree of disability or dependence in the daily activities of people who have suffered a stroke or other causes of neurological disability (Kasner SE. Lancet Neurol. 2006;7:603-12).

RIBOFLAVIN FOR THE TREATMENT OF GLUTAMATE EXCITOTOXICITY-ASSOCIATED DISEASES IN COMBINATION WITH OXALOACETATE

A first aspect of the present invention relates to riboflavin for use in the treatment of a disease associated with glutamate excitotoxicity in a subject in combination with oxaloacetate, wherein said treatment is a prophylactic treatment before the clinical onset of the disease or a therapeutic treatment after the clinical onset of the disease; and wherein combination is herein understood as the simultaneous or sequential administration of oxaloacetate and riboflavin to said subject.

In a related aspect it relates to a method of therapeutic or prophylactic treatment of a disease associated with glutamate excitotoxicity wherein such treatment comprises administering to a subject a therapeutically effective amount of riboflavin in combination with a therapeutically effective amount of oxaloacetate.

The term "riboflavin" as used herein refers to the molecule of chemical formula $C_{17}H_{20}N_4O_6$ identified with PubChem CID: 493570 and IUPAC name: 7,8-dimethyl-10-[(2S,3S,4R)-2,3,4,5-tetrahydroxypentyl]benzo[g]pteridine-2,4-dione), which is a water-soluble vitamin of the B group. It is also known as vitamin B2, lactoflavin, vitamin G, riboflavine or (-)-riboflavin. In the present invention "riboflavin" is also referred as CGB000592.

Riboflavin is present as an essential constituent of all living cells, and is therefore widely distributed in small amounts in foods. The major sources of riboflavin are milk, eggs, enriched cereals and grain, ice cream, liver, some lean meats, and green vegetables. Riboflavin is typically used for the treatment of ariboflavinosis (vitamin B2 deficiency). Like the other B vitamins, riboflavin is known to support energy production by aiding in the metabolising of fats, carbohydrates, and proteins. More specifically, riboflavin is the precursor of flavin mononucleotide (FMN, riboflavin monophosphate) and flavin adenine dinucleotide (FAD). The antioxidant activity of riboflavin is principally derived from its role as a precursor of FAD and the role of this cofactor in the production of the antioxidant reduced glutathione. Reduced glutathione is the cofactor of the selenium-containing glutathione peroxidases among other things. The glutathione peroxidases are major antioxidant enzymes. Reduced glutathione is generated by the FAD-containing enzyme glutathione reductase.

The term "riboflavin" as used herein further includes "derivatives thereof" such as the partially or totally protonated form, its pharmaceutically acceptable salts, and its esters. Riboflavin is commercially available as a salt, such as a sodium phosphate salt (e.g., riboflavin phosphate sodium dehydrate, riboflavin 5'-monophosphate sodium salt, riboflavin sodium phosphate, riboflavin 5'-monophosphate sodium salt hydrate, riboflavin 5'-phosphate sodium salt hydrate and riboflavin 5'-monophosphate sodium salt hydrate). The terms riboflavin and riboflavin sodium phosphate might be used herein interchangeably.

In a particular embodiment, the term "riboflavin sodium phosphate" as used herein may refer to the pharmaceutical composition defined in the EUROPEAN PHARMACOPOEIA 5.0 monography, as a mixture containing riboflavin 5'- (sodium hydrogen phosphate) as the main component and other riboflavin sodium monophosphates. It contains not less than 73.0 per cent and not more than 79.0 per cent of riboflavin ($C_{17}H_{20}N_4O_6$ ; $M_r$ 376.4), calculated with reference to the dried substance.

Under the first aspect of the present invention, riboflavin is used in combination with oxaloacetate. The term "oxaloacetate" as used herein refers to the deprotonated derivative of oxaloacetic acid with chemical formula $C_4H_3O_5$ identified with PubChem CID: 3260017 and IUPAC name: 3-carboxy-3-oxopropanoate. The term "oxaloacetate" as used herein also encompasses "derivatives thereof" such as the partially (conjugate base) or totally protonated form (oxaloacetic acid), its pharmaceutically acceptable salts, and its esters, such as diethyl oxaloacetate, and salts such as the disodium salt of diethyl oxaloacetate. Oxaloacetate is commercially available as a salt, for example the sodium salt. As a metabolite it is involved in various metabolic pathways such as the Krebs cycle or C-4 photosynthesis.

The term "disease associated with glutamate excitotoxicity" as used herein refers to those medical conditions where levels in blood are higher than 200 μM (Castillo J et al. Lancet 1997; 349:79-83). A common and standardized analytical method used for analyzing glutamate in blood or cerebrospinal fluid (CSF) is high-performance liquid chromatography (HPLC) (Castillo J et al. Lancet 1997; 349:79-83). Other well-known methods for the determination of analytes in blood or CSF will also be available to a person skilled in the art.

In a particular embodiment said disease is selected from the group consisting of cerebral ischaemia, traumatic brain injury, glaucoma and amyotrophic lateral sclerosis. Preferably, said disease is cerebral ischaemia, more preferably acute ischemic stroke.

Indeed, it has been shown in previous works of the inventors that levels of glutamate higher than 200 μM in blood or cerebrospinal fluid (CSF) in ischemic patients act as an important predictor of neuronal damage at 48 hours, with a sensitivity of 85% and a specificity of 97%. Moreover, high levels (>200 μM) of glutamate in plasma for at least 24 hours is associated with early neurological deterioration, whereas in patients who glutamate levels drop to normal values within the 6 hours from onset they will have a better outcome (Castillo J et al., Stroke. 1996; 27:1060-1065; Castillo J et al., Lancet. 1997; 349:79-83; Castillo J et al., Cerebrovasc Dis. 1999; 9:22-27; Castillo J et al., Stroke. 1995; 26:2035-2039).

The term "cerebral ischemia", also referred herein as "brain ischemia" or "cerebrovascular ischemia", is a condition in which there is insufficient blood flow to the brain to meet metabolic demand. This leads to poor oxygen supply or cerebral hypoxia and thus to the death of brain tissue or cerebral infarction also referred as "ischemic stroke". "Ischemic stroke" is a sub-type of stroke and is typically the result of the interruption of blood supply to the brain due to a occlusion of a cerebral artery. The terms "cerebral ischemia" and "ischemic stroke" may be used interchangeably in the present invention.

There are two types of cerebral ischemia: focal ischemia, which is confined to a specific region of the brain; and global ischemia, which encompasses wide areas of brain tissue. Typically, cerebral ischemia is characterized by the patient presenting one or more of the following symptoms: trouble with speaking and understanding, paralysis or numbness of the face, arm or leg, trouble with seeing in one or both eyes, headache and trouble with walking. To determine the type of stroke and the most appropriate treatment, the emergency team needs to evaluate the type of stroke and the areas of the brain affected by the stroke. They also need to rule out other possible causes of the symptoms, such as a brain tumor or a drug reaction. There are several tests that are generally used to determine the type of stroke: physical examination, blood tests (glucose levels, counting of blood cells, serum electrolytes such as sodium, potassium or calcium, cholesterol, total lipids, HDL, LDL or coagulation factors such as antithrombin III, protein C, protein S; factor VIII; activated Protein C resistance; specially relevant are coagulation factors and platelets determination), computerized tomography (CT) scan, magnetic resonance imaging (MRI), carotid ultrasound, cerebral angiogram and echocardiogram. For a review on diagnosis of ischemic stroke, see Am Fam Physician., 2015, 91(8):528-36.

The acute phase of ischemic stroke is referred herein as "acute ischemic stroke" defined as within 4 hours of onset.

Riboflavin and oxaloacetate may be administered in a sole composition or in separate compositions. Preferably, said composition(s) are pharmaceutical compositions. In a preferred embodiment, riboflavin and oxaloacetate are administered in a sole pharmaceutical composition. Details on suitable pharmaceutical compositions are provided below.

Administration can be simultaneous (at the same time) or sequential. "Sequential administration" as used herein refers to the administration of riboflavin or a composition comprising thereof shortly before or after administration of oxaloacetate or a composition comprising thereof "Shortly before or after" as used herein is understood as within an interval of 1 to 3 hours, preferably within 2 hours, more preferably within 1 hour or less, such as within 45 minutes, 30 minutes, 15 minutes or less. In a preferred embodiment, riboflavin and oxaloacetate administration is carried out simultaneously.

<u>Route of administration</u>

Said one or more pharmaceutical compositions are formulated to be compatible with its intended route of administration. Methods to accomplish the administration are known to those of ordinary skill in the art. This includes, for example, injections, by parenteral routes such as intravascular, intravenous, intraarterial, subcutaneous, intramuscular, intraperitoneal, intraventricular, intraepidural, or others as well as oral, nasal, ophthalmic, rectal, or topical. Sustained release administration is also specifically contemplated, by such means as depot injections or erodible implants. A preferred route of administration is intravascular administration, which is herein understood as the administration within a vessel or vessels and typically includes intravenous or intraarterial administration. Currently, the most preferred route of administration is intravenous administration.

It is currently preferred to administer riboflavin and/or oxaloacetate by intravenous injection, see Zempleni et al., Am J Clin Nutr 1996, 63:54-66 on the pharmacokinetics of intravenously administered riboflavin. Typically, riboflavin and oxaloacetate are administered simultaneously as an intravenous bolus injection. More than one dose can be administered, such as two or three doses. Preferably, a single dose is administered. Alternatively, riboflavin and/or oxaloacetate may be administered by infusion. The infusing step is typically repeated on a cyclic basis. In a preferred embodiment, riboflavin and oxaloacetate administration is carried out in a single dose simultaneously via intravenous bolus injection.

Dosage

The optimal dose will be selected according to the administration route, treatment regime and/or administration schedule, having regard to the existing toxicity and effectiveness data.

In a preferred embodiment the riboflavin and oxaloacetate combination is in a dosage capable of providing a neuroprotective effect in the absence of toxic effects, more specifically in the absence of toxic effects associated to succinate dehydrogenase inhibition.

No toxic or adverse reactions to riboflavin in humans have been identified. Because riboflavin is a water-soluble vitamin, typically excess amounts are excreted. According to the report of the Expert Group on Vitamins and Minerals (2003, pages 68-73, http://cot.food.gov.uk/sites/default/files/vitmin2003.pdf), there are insufficient data from human and animal studies to establish a Safe Upper Level for riboflavin, although the available data indicates that it is of low toxicity. No toxicological hazard has been identified for riboflavin from animal studies. The balance of evidence suggests that ingestion of riboflavin over prolonged periods of time is without harmful effects, even at many times the normal level of exposure. In a prophylactic study of migraine, doses of 400 mg riboflavin per day for at least 3 months were well tolerated (Schoenen et al., 1998). Based on previous supplementation studies in human, this group suggested, for guidance purposes only, supplemental intakes of 40 mg riboflavin/day (equivalent to 0.67 mg/kg body weight (bw) for a 60 kg adult) would be unlikely to result in adverse effects. This is in addition to riboflavin provided by the diet. Assuming a maximum dietary intake of 3.3 mg/day, a total daily intake of 43 mg riboflavin (equivalent to 0.72 mg/kg bw/day in a 60 kg adult) would not be expected to result in any adverse effects.

Accordingly, in the present invention, the dosage of riboflavin is not particularly restricted. Riboflavin may be administered in dosages up to 400 mg, preferably up to 300 mg, more preferably up to 200 mg, most preferably up to 100, 75, 50, 40, 30 or 25 mg. Particularly preferred ranges are from about 5 to about 50 mg, from about 10 to about 30 mg, or from about 10 to about 20 mg. Most preferred ranges are from about 5 to about 12 mg, from about 5 to about 15 mg and from about 5 to about 20 mg; preferred dosages being 5 mg, 10 mg, 12 mg and 20 mg.

Clinical dosages might also be provided as mg of active ingredient per kg of patient. In a particular embodiment, riboflavin is administered at a dosage from about 0.07 mg/kg to about 0.71 mg/kg, preferably from about 0.14 mg/kg to about 0.42 mg/kg, more preferably from about 0.14 mg/kg to about 0.28 mg/kg or from about 0.16 mg/kg to about 0.24 mg/kg. Preferred dosages are 0.16 mg/kg and 0.24 mg/kg.

Oxaloacetate may be administered at a dosage of below 4 mg/kg, preferably below 3 mg/kg, more preferably below 2.5 mg/kg, such as below 2.45 mg/kg or below 2.4 mg/kg. Preferred ranges are from about 3.9 mg/kg to about 2 mg/kg and from about 3.6 mg/kg to about 2.4 mg/kg.

In a preferred embodiment, riboflavin is administered at a dosage from about 0.07 mg/kg to about 0.71 mg/kg, preferably from about 0.14 mg/kg to about 0.42 mg/kg, more preferably from about 0.14 mg/kg to about 0.28 mg/kg, or from about 0.16 mg/kg to about 0.24 mg/kg; and oxaloacetate is administered at a dosage of below 4 mg/kg, preferably below 3 mg/kg, more preferably below 2.5 mg/kg, such as below 2.45 mg/kg or below 2.4 mg/kg. Most preferred ranges and dosages are as defined above.

Administration schedule

It is critical in the treatment of acute ischemic stroke to administer the treatment soon after the ischemia onset. Accordingly, in the present invention administration is generally carried out within the first twelve hours of ischemia symptom onset. Preferably, administration is carried out within the first six hours, more preferably within the first three or four hours. Most preferably within the first 2 hours, more preferably within 1 hour or less, such as within 45 minutes, 30 minutes, 15 minutes or less. In a preferred embodiment, administration is carried out minutes after symptom onset.

Furthermore, as above mentioned, the subject to be treated might be a patient diagnosed with acute ischemic stroke or a patient suspected of acute ischemic stroke. In a preferred embodiment, riboflavin and oxaloacetate are administered to a patient suspected of acute ischemic stroke within the first three hours from symptom onset.

A COMPOSITION COMPRISING RIBOFLAVIN AND OXALOACETATE

In a second aspect the present invention refers to a composition comprising riboflavin and oxaloacetate. In a particular embodiment, the present invention relates to a pharmaceutical composition comprising riboflavin, oxaloacetate and a pharmaceutically acceptable carrier, additive and/or excipient.

Pharmaceutically acceptable excipients include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. It is also preferred, that the preparation will contain a pharmaceutically acceptable excipient that serves as a stabilizer, particularly for peptide, protein or other like molecules if they are to be included in the vaccine composition. Examples of suitable carriers include, without limitation, pharmaceutical grades of dextrose, sucrose, lactose, trehalose, mannitol, sorbitol, inositol, dextran, and the like. Other suitable carriers include, starch, cellulose, sodium or calcium phosphates, citric acid, tartaric acid, glycine, high molecular weight polyethylene glycols (PEGs), and combination thereof. A thorough discussion of pharmaceutically acceptable excipients, additives and carriers is available in Remington's Pharmaceutical Sciences (22nd edition, 2012).

Said composition, preferably a pharmaceutical composition, comprises riboflavin and oxaloacetate in the dosages defined in the first aspect of the invention and it is administered according to the administration route and schedules defined under the first aspect of the invention. Further details and preferred embodiments on the dosage, route and schedules of administration of said composition are also provided under the first aspect of the invention.

Preferably, said composition is in a form suitable for intravascular administration. In a preferred embodiment, said composition is an aqueous composition, more preferably a stable aqueous composition. As used herein, a "stable composition" may refer to a formulation in which the active ingredient therein essentially retains its physical stability and/or chemical stability and/or biological activity upon storage.

Typically, riboflavin and/or oxaloacetate are formulated in saline solution (0.9% of NaCl, preferably pH adjusted to 7.4) or 5% glucose solution. A commercially available pharmaceutical form of riboflavin sodium phosphate as a solution for injection is "Vitamin B2 Streuli®" (Streuli®, Switzerland).

Some embodiments of such compositions may be provided by lyophilised formulations. Said lyophilised formulations can be reconstituted and diluted to give a composition of this invention in the form of a solution ready for intravascular injection. The actual amounts of reconstituting fluid are not limiting features of embodiments of this invention. By way of illustrations, but not as limitations, embodiments of lyophilised formulations according to this invention are reconstituted with a volume of water. Most of such volumes do not exceed about 20 ml, with preferred volumes being in the range from about 1 ml to about 15 ml, more preferably in the range from about 1 ml to about 10 ml, and even more preferably in the range from about 3 ml to about 8 ml, and even more preferably about 5 ml.

Reconstituted embodiments of the present invention can further be diluted if so desired, with this further dilution not being a limitation of the present invention. This further dilution is preferably carried out with an aqueous system which is usually 0.9% sodium chloride or 5% glucose. The reconstituted solution will be diluted depending on the concentration in the reconstituted solution and the desired concentration in the diluted solution.

The present invention further relates to said composition for use as defined in the first aspect of the invention.

## A KIT OF PARTS COMPRISING RIBOFLAVIN AND OXALOACETATE

A third aspect of the invention refers to a kit of parts comprising riboflavin and oxaloacetate, together with instructions for use in the treatment of a disease involving the simultaneous or sequential administration of both active ingredients.

The term "kit of parts" as used herein refers to a combined preparation wherein the active ingredients are physically separated for use in a combined therapy by simultaneous administration or sequential administration to the patient.

Typically, the two active ingredients are provided formulated as a pharmaceutical composition and provided in two separate recipients. Preferably, said compositions are as defined in the previous aspects of the invention, for use in a method of treatment as defined in the previous aspects of the invention, preferably in the treatment of a disease associated with glutamate excitotoxicity, preferably in the treatment of acute ischemic stroke.

## RIBOFLAVIN FOR USE IN THE THERAPEUTIC TREATMENT OF ACUTE ISCHEMIC STROKE

A forth aspect of the present invention relates to riboflavin for use in the therapeutic treatment of acute ischemic stroke by intravascular administration to a subject suffering or suspected to suffer from acute ischemic stroke within the first twelve hours of symptom onset.

Riboflavin has been defined under the first aspect of the invention. Details and preferred embodiments on the dosage, route and schedules of administration of riboflavin are provided under the previous aspects of the invention.

In particular, riboflavin administration may be carried out as a single administration. Said administration is preferably a single intravascular bolus injection.

In the present invention, the dosage of riboflavin is not particularly restricted. Riboflavin may be administered in dosages up to 400 mg, preferably up to 300 mg, more preferably up to 200 mg, most preferably up to 100, 75, 50, 40, 30 or 25 mg. Particularly preferred ranges are from about 5 to about 50 mg, from about 10 to about 30 mg, or from about 10 to about 20 mg. Most preferred ranges are from about 5 to about 12 mg, from about 5 to about 15 mg and from about 5 to about 20 mg; preferred dosages being 5 mg, 10 mg, 12 mg and 20 mg.

Clinical dosages might also be provided as mg of active ingredient per kg of patient. In a particular embodiment, riboflavin is administered at a dosage from about 0.07 mg/kg to about 0.71 mg/kg, preferably from about 0.14 mg/kg to about 0.42 mg/kg, more preferably from about 0.14 mg/kg to about 0.28 mg/kg or from about 0.16 mg/kg to about 0.24 mg/kg. Preferred dosages are 0.16 mg/kg and 0.24 mg/kg.

Riboflavin may be administered within the first six hours of symptom onset, preferably within the first three or four hours. Most preferably within the first 2 hours, more preferably within 1 hour or less, such as within 45 minutes, 30 minutes, 15 minutes or less. In a preferred embodiment, administration is carried out minutes after symptom onset.

In a fifth aspect, the present invention further provides a pharmaceutical composition comprising riboflavin, and a pharmaceutically acceptable carrier, additive or excipient for use according to the forth aspect of the invention.

Pharmaceutically acceptable excipients include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. It is also preferred, that the preparation will contain a pharmaceutically acceptable excipient that serves as a stabilizer, particularly for peptide, protein or other like molecules if they are to be included in the vaccine composition. Examples of suitable carriers include, without limitation, pharmaceutical grades of dextrose, sucrose, lactose, trehalose, mannitol, sorbitol, inositol, dextran, and the like. Other suitable carriers include, starch, cellulose, sodium or calcium phosphates, citric acid, tartaric acid, glycine, high molecular weight polyethylene glycols (PEGs), and combination thereof. A thorough discussion of pharmaceutically acceptable excipients, additives and carriers is available in Remington's Pharmaceutical Sciences (22nd edition, 2012).

Preferably, said pharmaceutical composition is in a form suitable for intravascular administration. In a preferred embodiment, said composition is an aqueous composition, more preferably a stable aqueous composition. As used herein, a "stable composition" may refer to a formulation in which the active ingredient therein essentially retains its physical stability and/or chemical stability and/or biological activity upon storage.

Typically, riboflavin is formulated in saline solution (0.9% of NaCl, preferably pH adjusted to 7.4) or 5% glucose solution. A commercially available pharmaceutical form of riboflavin sodium phosphate as a solution for injection is "Vitamin B2 Streuli®" (Streuli®, Switzerland).

Some embodiments of such compositions may be provided by lyophilised formulations. Said lyophilised formulations can be reconstituted and diluted to give a composition of this invention in the form of a solution ready for intravascular injection. The actual amounts of reconstituting fluid are not limiting features of embodiments of this invention. By way of illustrations, but not as limitations, embodiments of lyophilised formulations according to this invention are reconstituted with a volume of water. Most of such volumes do not exceed about 20 ml, with preferred volumes being in the range from about 1 ml to about 15 ml, more preferably in the range from about 1 ml to about 10 ml, and even more preferably in the range from about 3 ml to about 8 ml, and even more preferably about 5 ml.

Reconstituted embodiments of the present invention can further be diluted if so desired, with this further dilution not being a limitation of the present invention. This further dilution is preferably carried out with an aqueous system which is usually 0.9% sodium chloride or 5% glucose. The reconstituted solution will be diluted depending on the concentration in the reconstituted solution and the desired concentration in the diluted solution.

Moreover, said pharmaceutical composition, comprises riboflavin in the dosages defined in the first aspect of the invention and it is administered according to the administration route and schedules defined under the previous aspects of the invention. Further details and preferred embodiments on a pharmaceutical composition comprising riboflavin, the dosage, route and schedules of administration and use of said composition in acute ischemic stroke are also provided under the previous aspects of the invention.

It will be understood that particular embodiments described in the Examples are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference.

The use of the word "a" or "an" may mean "one," but it is also consistent with the meaning of "one or more", "at least one," and "one or more than one."

Throughout this application, the term "about" means the indicated value ± 10% of its value, preferably the indicated value ± 5% of its value, more preferably the indicated value ± 2% of its value, most preferably the term "about" means exactly the indicated value (± 0%).

## EXAMPLES

### Example 1: Materials and Methods

#### 1.1 *In vitro* screening of known drugs for their ability to reduce glutamate formation

The Prestwick chemical library® (1120 compounds) was screened for the identification of compounds having the ability to reduce the formation of glutamate in presence of glutamate oxaloacetate transaminase (GOT). Notably, inhibition of glutamate formation was evaluated by use of a photometric kit (Glutamic Oxaloacetic Transaminase Activity Assay Kit from Abcam, ref. ab105135). The test was carried out with recombinant human GOT (from Abcam, ref. ab99147). Compounds were tested at a final concentration of $10^{-4}$ M and the assay conditions were fixed following manufacturer's specifications. Hits were selected and confirmed in a new dose-response assay.

#### 1.2 *In vitro* determination of succinate dehydrogenase (SDH) inhibition

To evaluate possible toxic effects on SDH, a secondary assay was set up, using Complex II Enzyme Activity Microplate Assay (from Abcam, ref. ab109908), based on the detection of 2,6-diclorophenolindophenol (DCPID), blue, which is reduced in contact with Ubiquinol, turning colorless and involving the measurement of a decrease in absorbance at 595 nm. The enzyme was extracted from HepG2 cells (ATCC, ref. HB-8065), following manufacturer's instructions. Hit compounds were tested at 2 concentrations: $10^{-5}$ M and $10^{-4}$ M. Hits were confirmed in a new dose-response assay, using hit compounds provided from a new supplier: (-)-Riboflavin (CBG000592), SIGMA, ref. R4500 ; R-(-)-Apomorphine hydrochloride hemihydrate (CBG000083, SIGMA, ref. A4393; Nifedipine (CBG000023), SIGMA, ref. N7634; (-)-3-(3,4-Dihydroxyphenyl)-2-methyl-L-alanine sesquihydrate (a.k.a. Methyldopa L-) (CBG000361), SIGMA, ref. 857416; Dobutamine hydrochloride (CBG000332), SIGMA, ref. D0676; Doxycycline hyclate (CBG000814), SIGMA, ref. D9891.

#### 1.3 *In vitro* determination of optimal riboflavin and oxaloacetate dose combination

Crossed dose-response curves of oxaloacetate and riboflavin were conducted to find the optimal dose combination at which the maximum level of inhibition obtained by oxaloacetate (alone) is reached, at concentrations that do not affect succinate dehydrogenase (SDH). The assay was based in Glutamic Oxaloacetic Transaminase (GOT) Activity Assay Kit (from Abcam, ref. ab105135).

#### 1.4 Animals

Experimental protocols were approved by the local Animal Care Committee according to the European Union (EU) rules (86/609/CEE, 2003/65/CE and 2010/63/EU). Male Sprague-Dawley rats (Harlan Laboratories, Barcelona, Spain) with an average weight of 350 g were used. Rats were watered and fed ad libitum.

When undergoing surgery, anesthesia was induced by inhalation of 5% sevoflurane (Abbott laboratories, IL, USA) in a nitrous oxide/oxygen mixture (70/30). Rectal temperature was maintained at 37±0.5 °C by using a feedback-controlled heating pad. Glucose levels were analyzed before surgery (ranging from 180 to 220 mg/dl).

#### 1.5 *In vivo* analysis of the effect of riboflavin in healthy animals

The dose-response effect of riboflavin on blood glutamate lowering was determined in healthy animals. Three (3) doses of riboflavin were tested (i.v.), 1, 10 and 50 mg/Kg (weight of animal). As control group was used animals treated with saline (0.9% of NaCl, pH adjusted to 7.4), and as positive control group, animals treated with oxaloacetate 35 mg/Kg. This dose of oxaloacetate was chosen based on our previous studies where we demonstrated that an i.v. dose of 35 mg/Kg (weight of animal) of oxaloacetate induced a significant reduction in serum glutamate concentration (Campos F. et al., J Cereb Blood Flow Metab., 2011, 31:1378-1386). A total of 6 animals per group were included in this study. Levels of blood glutamate were determined in basal condition (before treatment administration), 30 min, 2, 4 and 6 hours after treatments administration.

### 1.6 *In vivo* analysis of the effect of riboflavin alone or in combination with oxaloacetate in ischemic model animals

With the aim to determine if the reduction on blood glutamate levels induced by riboflavin led to a reduction on infarct damage and an improvement of neurological deficit, the effective dose selected (1 mg/Kg) in the previous dose-response study was tested in ischemic animals.

Four (4) experimental groups were performed: 1) a control group treated with saline (0.9% of NaCl, pH adjusted to 7.4), 2) a group treated with riboflavin 1 mg/Kg, 3) a group treated with riboflavin 1 mg/Kg in combination with dose of oxaloacetate a non-effective (15 mg/Kg) and 4) a positive control group treated with oxaloacetate 35 mg/Kg. Dose of 15 mg/Kg as non-effective dose of oxaloacetate was chosen based on our previous studies (Campos F. et al., J Cereb Blood Flow Metab., 2011, 31:1378-1386; and Perez-Mato M et al., Cell Death Dis., 2014, 5:e992). Treatments were administered after artery reperfusion (45 min after occlusion). A total of 10 animals per group were included in this study.

Levels of blood glutamate were determined in basal conditions (before ischemic surgery), 1, 3 and 24 hours after artery occlusion. Infarct volume analysis were evaluated during the occlusion (30 min after occlusion), and 24h and 7 days after ischemia induction. Infarct volume determined at 30 min after artery occlusion (analyzed by diffusion weighted imaging, DWI) (Pérez-Mato, M., et al., Cell Death Dis., 2014, 5:e992) was determined before treatments administration to confirm that all animals included in the study were submitted to the same ischemic insult. These values were used as basal infarct volume.

Neurological deficit was evaluated by means of cylinder test and Bederson test (described below) performed 1 day before surgery (used as basal conditions) and 24h and 7 days after ischemia.

### 1.7 Riboflavin and oxaloacetate formulations used in *in vivo* models

In all animals treated, healthy animals and ischemic animals, a final volume of 1 mL of each treatment was administered to treat the animals. With this purpose, dose of 35mg/Kg (average weight of animals: 350 g) of oxaloacetate (Ref. 171263, Sigma) was prepared in a saline solution (0.9% of NaCl, pH adjusted to 7.4) at a final concentration of 12.25 mg/mL. Dose of 15 mg/Kg of oxaloacetate was prepared in saline solution (0.9% of NaCl, pH adjusted to 7.4) at a final concentration of 5.25 mg/mL. Dose of 1 mg/Kg of riboflavin (Ref. 4500, Sigma) initially dissolved in a minimal amount of dimethyl sulfoxide (DMSO) and then dissolved in a saline solution (0.9% of NaCl, pH adjusted to 7.4) at a final concentration of 0.35 mg/mL. A volume of 1 mL of saline was injected in control animals. For doses of 10 and 50 mg/Kg in a minimal amount of dimethyl sulfoxide (DMSO) and dissolved in a saline solution (0.9% of NaCl, pH adjusted to 7.4) at a final concentration of 3.5 mg/mL and 17.5 mg/mL respectively.

### 1.8 Serum glutamate analysis

Serum samples were collected in test tubes, centrifuged at 3000 r.p.m. for 7 min, serum was removed and immediately frozen and stored at -80 °C. Serum glutamate concentration was determined by means of Glutamate Assay Kit (Abnova, Taipei City, Taiwan) following the manufacturer's technical specifications.

### 1.9 Dose translation rats to humans

The amounts of oxaloacetate administered to rats were extrapolated to a human dose as described in Reagan-Shaw et al., "Dose translation from animal to human studies revisited", The FASEB Journal, 2007, 22, 659-661, using the equation:

$$\text{Dose in humans (mg/kg)} = \text{Dose in animal (mg/kg)}*(\text{km animal/km human})$$

Using the Km values shown in the table below:

| Species | Weight (kg) | BSA ($m^2$) | $K_m$ factor |
|---|---|---|---|
| Human | | | |
| Adult | 60 | 1.6 | 37 |
| Child | 20 | 0.8 | 25 |
| Baboon | 12 | 0.6 | 20 |
| Dog | 10 | 0.5 | 20 |
| Monkey | 3 | 0.24 | 12 |

(continued)

| Species | Weight (kg) | BSA ($m^2$) | $K_m$ factor |
|---|---|---|---|
| Rabbit | 1.8 | 0.15 | 12 |
| Guinea pig | 0.4 | 0.05 | 8 |
| Rat | 0.15 | 0.025 | 6 |
| Hamster | 0.08 | 0.02 | 5 |
| Mouse | 0.02 | 0.007 | 3 |

Values based on data from FDA Draft Guidelines (7). To convert dose in mg/kg to dose in mg/$m^2$, multiply by $K_m$ value.

### 1.10 Surgical procedures

Transient focal ischemia was induced in rats by using transient Middle Cerebral Arterial Occlusion (MCAO) following the surgical procedures previously described (Campos F. et al., J Cereb Blood Flow Metab., 2011, 31:1378-1386). In brief, under an operating microscope, the left common, external and internal carotid arteries were dissected from connective tissue through a midline neck incision. The left external carotid artery and pterygopalatine artery of the internal carotid artery were separated and ligated by 5-0 silk sutures. A 23-mm segment of 3-0 nylon monofilament suture with the tip rounded by heat was inserted into the stump of the left common carotid artery and advanced into the internal carotid artery to 20 mm from the bifurcation to occlude the origin of the MCA. The suture was removed after 45 min of occlusion. A laser-Doppler flow probe (tip diameter 1 mm) attached to a flowmeter (PeriFlux 5000; Perimed AB, Stockholm, Sweden) was located over the thinned skull in the MCA territory (4 mm lateral to bregma) to obtain a continuous measure of relative cerebral flow during the experiment. Only animals with a cerebral serum flow reduction of over 70% and with reperfusion after occlusion were included in the study. Experimental procedures were performed following five criteria derived from the Stroke Therapy Academic Industry Roundtable (STAIR) group guidelines for preclinical evaluation of stroke therapeutics Recommendations for standards regarding preclinical neuroprotective and restorative drug development (Albers GW et al., Stroke, 1999, 30: 2752-2758; and Philip M et al., Stroke, 2009, 40:577-581): (1) cerebral serum flow was measured to confirm the vascular occlusion as an index of the reliability of the ischemic model; (2) animals were randomly assigned to treatment groups of the study; (3) researchers were blinded to treatment administration; (4) researchers were blinded to treatments during outcome assessment; and (5) temperature was controlled during the ischemic period.

### 1.11 Magnetic resonance imaging protocol

Infarct volume, also referred herein as infarct size, was assessed by means of magnetic resonance imaging (MRI). MRI studies were conducted on a 9.4-T horizontal bore magnet (Bruker BioSpin, Ettligen, Germany) with 12 cm wide actively shielded gradient coils (440 mT/m). Radiofrequency (RF) transmission was achieved with a birdcage volume resonator; signal was detected using a four-element arrayed surface coil, positioned over the head of the animal, which was fixed with a teeth bar, earplugs and adhesive tape. Transmission and reception coils were actively decoupled from each other. Gradient-echo pilot scans were performed at the beginning of each imaging session for accurate positioning of the animal inside the magnet bore. Apparent diffusion coefficient (ADC) maps were acquired during MCA occlusion (30 min after the onset of ischemia) using a spin-echo echo-planar imaging sequence with the following acquisition parameters: field-of-view 19.2 $\times$ 19.2 mm2, image matrix 128 $\times$ 128 (in-plane resolution 0.15 mm/pixel), 14 consecutive slices of 1 mm thickness, repetition time=4 s, echo time=30 ms and diffusion b values: 0, 100, 300, 600, 800, 1000 and 1400 s/mm2. T2-weighted image was acquired 24 h and 7 days after the onset of ischemia.

### 1.12 Image analysis

All images were processed and maps were constructed with ImageJ (Rasband WS, ImageJ, US National Institutes of Health, Bethesda, MD, USA, http://rsb.info.nih. gov/ij/, 1997-2009). Infarct volumes were determined from (ADC maps) and T2-weighted images by manually selecting areas of reduced ADC values or hyperintense T2 signal by a researcher blinded to the animal protocols.

### 1.13 Sensorimotor test

Sensorimotor deficits were assessed using the cylinder test (Schallert T et al., Neuropharmacology, 2000, 39:777-787 and Bederson test (Schaar KL et al., Exp Transl Stroke Med., 2010, 2:13).

Cylinder test

Cylinder test consists of evaluation of asymmetry of limbs during the exploratory activity. For this test, the animal was put in a cylinder of transparent base of 20 cm diameter. A video camera is located under this transparent cylinder for recording the vertical exploratory movement of the animal with anterior limbs during 2-10 min, depending on movement grade. For recording analysis, the VirtualDub software (http://www.virtualdub.org/) was used. Analyzed behaviors were as follows: number of times that the animal touches the cylinder wall and independent use of each limb in contact with the cylinder wall in each upward movement. Laterality index was calculated (the number of times that the animal touches the cylinder with the right leg during the ascendant movement by the number of times that the animals touch with each leg). This index is close to 0.5 for healthy animals and tends to be 0 or 1 for animals that have a preferential use of the left or the right paw, respectively. Somatosensory tests were performed 1 day before MCAO and on day 7 after ischemia during the darkness cycle.

Bederson test

Bederson scale is a global neurological assessment that was developed to measure neurological impairments following stroke. Tests include forelimb flexion, resistance to lateral push and circling behavior. A grading scale of 0-8 is used to assess behavioral deficits after cerebral damage. Zero value represents animal without neurological deficit and 8 with high neurological deficit. Somatosensory tests were performed 1 day before MCAO and on day 7 after ischemia during the darkness cycle.

**1.14 Statistical analysis**

Results were expressed as mean$\pm$S.E.M. Statistical analyses were performed using one-way ANOVA followed by a Bonferroni post hoc analysis to determine between-group differences. A P-value <0.05 was considered as statistically significant. The statistical analysis was conducted using PASW Statistics 18 for Mac (SPSS Inc., Chicago, CA, USA).

**Example 2: In vitro screening of known drugs for their ability to inhibit glutamate formation**

Drugs included in the Prestwick Chemical Library® (1120 compounds) were selected to analyze their ability to modify GOT and SDH activity. Among the 1120 drugs tested, 6 of them showed significant inhibition of GOT activity (Table 1). Five (5) of these 6 compounds showed a concentration-dependent effect, and thus it was possible to determinate the $IC_{50}$ value. Based on inhibition of glutamate formation in the confirmatory assay, the most potent one, CBG000592 ($IC_{50}$: 1.96 $\mu$M) was selected as a promising glutamate scavenger drug.

**Table1:** Effect in GOT activity, measured in inhibition of glutamate formation and IC50, of 6 hits from the High-Throughput Screening (HTS) assay. Data obtained from confirmatory assay.

| **Compound** | **Maximum of inhibition of glutamate formation in confirmatory assay (%)** | **IC50($\mu$M) in confirmatory assay** |
|---|---|---|
| CBG000083 | 56,5 | 58,1 |
| CBG000023 | 48 | --- |
| CBG000361 | 38,5 | 117,1 |
| CBG000332 | 35,5 | 81,7 |
| CBG000592 | 63 | 1,96 |
| CBG000814 | 32 | 15,1 |

CBG000592 corresponded to the known drug riboflavin (vitamin B2). To determine if riboflavin would induce potential toxic effects by inhibition of SDH activity (as was previously observed with oxaloacetate at therapeutic doses, see Staples J. J Comp Physiol B., 2010,180:775-783), it was determined in an in vitro assay the effect of riboflavin in SDH activity (Figure 1). Analysis of dose-response curves of riboflavin on GOT and SDH activities was determined in comparison with oxaloacetate. The data obtained showed that, oxaloacetate presents a lower affinity for GOT than for SDH ($IC_{50}$: 0.13 mM (GOT) and 1.8 $\mu$M (SDH)), while riboflavin shows higher affinity for GOT than for SDH ($IC_{50}$: 1.9 $\mu$M *vs* 10.8 $\mu$M). Accordingly, oxaloacetate doses effective *in vitro* in modulating GOT (i.e. reducing glutamate formation) are shown to strongly inhibit the activity of SDH and thus expected to be toxic, as SDH is involved in the Krebs cycle

and the electron transport chain. This implies that riboflavin has a better therapeutic range.

Interestingly, when riboflavin was tested in combination with minimal amounts of oxaloacetate (0.0026 mM), as shown in Figure 2, the effect of riboflavin in increasing glutamate metabolism was potentiated, thereby decreasing blood glutamate concentration..

Doses of 2.6 x 10e-6 M of oxaloacetate and 10e-5 M of riboflavin were shown to provide synergistic effects in the modulation of GOT and the reduction of glutamate formation as shown in Figure 2. Indeed, Figure 2 shows that the combined use at said doses inhibits the formation of glutamate 65% more than the same doses of oxaloacetate when administered alone. At these concentrations of oxaloacetate, SDH is inhibited in very low percentages which are expected to not negatively impact cell viability (see Figure Ibis). As per the dose-response curve of oxaloacetate shown in Fig.1bis, these concentrations of oxaloacetate as single agent are non-effective in modulating GOT.

**Example 3: Analysis of riboflavin on blood glutamate levels in healthy animals**

Administration of saline in control group of healthy animals did not modify the blood glutamate levels during the period of study (6 hours). The 3 doses (1, 10 and 50 mg/Kg) of riboflavin tested, caused a significant ($p<0,05$) reduction of blood glutamate levels 30min, 2,4 and 6h after administration. The percentage of blood glutamate lowering was about 25 % (respect to basal levels), at 30 min, about 50 % at 2h, 60 % at 4h and 50 % at 6. No significant deference between the 3 doses on blood glutamate reduction was observed (Figure 3). Oxaloacetate, used as positive control of blood glutamate reduction, caused a reduction ($p<0,05$) of blood glutamate levels at 2, 4 and 6 hours after administration, however no effect was observed at 30 min. Percentage of glutamate levels reduction induced by oxaloacetate at 2, 4 and 6 hours was not higher than 40 % in all time-points. These oxaloacetate results are in line with our previous studies (Campos F. et al., J Cereb Blood Flow Metab., 2011, 31:1378-1386; and Perez-Mato M et al., Cell Death Dis., 2014, 5:e992).

Comparative analysis of the effect of riboflavin and oxaloacetate on glutamate reduction showed that the effect obtained with riboflavin is earlier and higher than the comparison drug (oxaloacetate), which is the gold standard approach for reducing increased glutamate levels after brain ischemia.

Due to the fact that there were no significant differences between the three doses of riboflavin tested, it was decided to select the lower dose (1 mg/Kg) to determine its protective effect in an ischemic animal model.

In light of the data obtained in this study, and extrapolating to the human dose using the equation and conversion table mentioned above, the dose of riboflavin for adult human patients is approximately 0.162 mg/kg.

$$\text{HED (mg/kg)} = 1 \text{ mg/kg x } 6/37 = 0.162 \text{ mg/kg}$$

Considering an average weight of 60-70 kg for an adult human, a suitable amount of riboflavin for administration to a human adult patient is approximately 9.72-11.34 mg.

If the patient was a child weighting 20kg the calculation would be the following

$$\text{HED (mg/kg)} = 1 \text{ mg/kg x } 6/25 = 0.24 \text{ mg/kg}$$

Considering an average weight of 20 kg for a human child, a suitable amount of riboflavin for administration to a human adult patient is approximately 4.8 mg.

Example 4: Analysis of riboflavin in an ischemic animal model

Cerebral artery occlusion during 45 min did not alter the basal levels of blood glutamate concentration (Figure 4). In sham-operated rats (having undergone a faked surgical intervention that omits the step thought to be therapeutically necessary) in which MCA was not occluded, no changes in plasma glutamate concentration were detected (data not shown). Riboflavin 1 mg/Kg and oxaloacetate 35 mg/Kg reduced about 30% the blood glutamate levels at 2 hours after their administration ($p<0.05$), respect to the basal levels. In case the treatment with riboflavin 1 mg/Kg in combination with a low amount of oxaloacetate (15 mg/Kg), the lowering to glutamate at 2 hours was also higher (reduction of 50%) than the other two treatments alone ($p<001$). The effect to this combination was also earlier, as the reduction was observed immediately (15 min) after their administration. Twenty four hours after treatments administration, blood glutamate concentrations come back to normal values. This results demonstrate that the reposition drug riboflavin has similar effect of blood glutamate lowering than oxaloacetate, and it effect are potentiated in combination with a minimal amount of oxaloacetate, in agreement with the previous in vitro assays.

To demonstrate that the reduction on blood glutamate levels observed after riboflavin administration led to a neuroprotective effect on the animal model of cerebral ischemia, the infarct volumes of treated versus control animals were compared. As shown in Figure 5, riboflavin or oxaloacetate caused a significant ($p<0.001$) decrease of lesion size measured at 24h and 7 days after ischemia. In line with the effect observed on blood glutamate, infarct reduction was higher when riboflavin 1 mg/Kg was administered in combination with of oxaloacetate (15 mg/Kg).

Diffusion-weighted images (DWI) show that infarct volumes were similar for both experimental groups before treatment.

Results indicated as absolute infarct volume ($mm^3$) or lesion volume relative to the basal (%), showed similar effect regarding the infarct volume reduction.

Somatosensory tests (cylinder test and Bederson test), which are considered to be important end point assays of drug screening in stroke, confirmed that reduction in infarct volume observed with each of the three treatments was associated with a better neurological outcome measured 7 days after ischemia (Figure 6).

A 15 mg/kg dose of oxaloacetate was selected for the combination assay as it was previously shown by the inventors to be non-effective, no changes on blood glutamate levels were observed within the first 3 hours after administration in healthy animals (Campos F. et al., J Cereb Blood Flow Metab., 2011, 31:1378-1386). Because oxaloacetate has shown a lower affinity for GOT than for SDH (see Fig.1), the inventors have suggested that oxaloacetate at non-effective levels (i.e., levels which do not activate GOT) should not affect SDH, and thus have no toxic effects derived from the inhibition of SDH.

In light of the data obtained in this study, and extrapolating to the human dose using the equation and conversion table mentioned above, a suitable dose of oxaloacetate to be used in combination with riboflavin for adult human patients is approximately 0.162 mg/kg.

$$\text{HED (mg/kg)} = 15 \text{ mg/kg x } 6/37 = 2.43 \text{ mg/kg}$$

Considering an average weight of 60-70 kg for an adult human, a suitable amount of riboflavin for administration to a human adult patient is approximately 145.8-170.1 mg.

If the patient was a child weighting 20kg the calculation would be the following

$$\text{HED (mg/kg)} = 15 \text{ mg/kg x } 6/25 = 3.6 \text{ mg/kg}$$

Considering an average weight of 20 kg for a human child, a suitable amount of riboflavin for administration to a human adult patient is approximately 72 mg.

Accordingly, levels of oxaloacetate below 15 mg/kg in rats (corresponding to 2.43 mg/kg in a human adult) are not expected to be linked to toxicity by inhibition of SDH.

**Example 5: "Randomized clinical trial with two parallel groups, double-blind, placebo-controlled trial to investigate whether administration of CBG000592 (riboflavin) in patients with acute ischemic stroke causes a reduction of glutamate-mediated excitotoxicity"**

Objectives of the trial:

i. Main objective of the trial: Assess whether CBG000592 administration in patients with acute ischemic stroke induces a reduction of serum glutamate concentration.

ii. Secondary objectives of the trial:

1. Study whether patients with acute ischemic stroke and treated with CBG000592 have lower average stay than those who receive placebo.
2. Study if patients with acute ischemic stroke and were treated with CBG000592 have a higher percentage of clinical improvement (basal-high) than those who receive placebo.
3. Consider whether patients with acute ischemic stroke and treated with CBG000592 have a better functional outcome than those who receive placebo.
4. Investigate the variations of serum glutamate levels among acute ischemic stroke patients treated with

CBG000592 or placebo.
5. Explore whether the prognosis of patients receiving CBG00592 and have no stroke is not worse than those treated with placebo.
6. Assess if CBG000592 administration in patients with clinical suspicion of stroke, administered within the first three hours of onset is safe.

Patients

i. Principal inclusion criteria:

- Patients older than 18 years, both men and women.
- Patient or legal representative able to understand and sign the informed consent.
- Patients with suspected stroke within 3 hours of onset.

ii. Principal exclusion criteria:

- Women of childbearing age, with potential for pregnancy or breastfeeding.
- Patients with a score 2 point 1a in the NIHSS scale.
- Pre-stroke modified Rankin scale >2.
- Inability to obtain prior image tests, such as computed tomography (CT) or magnetic resonance imaging (MRI), needed for the study.
- Existence of previous disorders that can affect the movement or behavior of the patient, and may thus interfere with the interpretation of neurological scales.
- Treatment with probenecid, tricyclic antidepressants, phenothiazines, streptomycin, erythromycin, tirotricina, tetraciclines and carbomycin, at the time of inclusion.

Treatment

A single intravenous bolus injection of riboflavin sodium phosphate ("Vitamin B2 Streuli®", Streuli®, Switzerland) at a dosage of 20 mg is given to patients with suspected stroke within 3 hours of onset. The placebo arm is administered a saline solution (0.9 NaCl) for injection.

End points

i. Primary end point:

- Definition: Difference between serum glutamate concentration from basal levels (prior to drug/placebo administration) and levels at 3 $\pm$ 1 and 6 $\pm$ 1 hour from administration.
- Timepoint(s) of evaluation: Baseline (prior to drug/placebo administration) and at 3$\pm$1 and at 6$\pm$1 hour from administration

ii. Secondary end point(s):

- To study the average length of stay in patients with acute ischemic stroke and establishing whether there is a difference between the two treatment arms.
- To study the rate of clinical improvement in patients with acute ischemic stroke: clinical improvement according to the formula: ($NIHSS_{basal}$-$NIHSS_{discharge}$) / $NIHSS_{basal}$ x 100 and compared between the two treatment arms.
- To study the functional outcome in patients with acute ischemic stroke: modified Rankin scale at 90 days, between two treatment arms.
- Variations of serum glutamate curves in patients with acute ischemic stroke between the two treatment arms: all concentrations of serum glutamate.
- To explore the prognosis of patients without stroke, evaluating modified Rankin scale at 90 days.
- Safety management: assess whether administration of CBG000592 in patients suspected of ischemic stroke administered within the first 3 hours of onset is safe.

iii. Timepoint(s) of evaluation of the secondary point(s):

- Average length of stay in patients with acute ischemic stroke is determined at discharge.
- Rate of clinical improvement in patients with acute ischemic stroke is determined at discharge.
- Functional outcome in patients with acute ischemic stroke: modified Rankin scale is determined at 90 days.
- Variations of serum glutamate curves in patients with acute ischemic stroke between two branches: all concentrations of serum glutamate is determined when the patient arrive to the hospital, at 3, 6, 12 and 24h after stroke onset.
- Prognosis of patients without stroke: modified Rankin scale is determined at 90 days.
- Safety management: adverse events are measured throughout the study.

## Claims

**1.** Riboflavin for use in the treatment of a disease associated with glutamate excitotoxicity in a subject, in combination with oxaloacetate, wherein said treatment is a prophylactic treatment before the clinical onset of the disease or a therapeutic treatment after the clinical onset of the disease; and wherein combination is herein understood as the simultaneous or sequential administration of oxaloacetate and riboflavin to said subject.

**2.** Riboflavin for use according to claim 1, wherein said disease is selected from the group consisting of cerebral ischaemia, traumatic brain injury, glaucoma and amyotrophic lateral sclerosis.

**3.** Riboflavin for use according to any of claims 1 or 2, wherein said disease is cerebral ischaemia, preferably acute ischemic stroke.

**4.** Riboflavin for use according to any of claims 1 to 3, wherein riboflavin and oxaloacetate are administered in a sole pharmaceutical composition or in separate pharmaceutical compositions.

**5.** Riboflavin for use according to any of claims 1 to 4, wherein riboflavin and oxaloacetate administration is carried out simultaneously, preferably in a single dose via intravascular bolus injection.

**6.** Riboflavin for use according to any of claims 1 to 5, wherein the combination is in a dosage capable of providing a neuroprotective effect in the absence of toxic effects associated to succinate dehydrogenase inhibition.

**7.** Riboflavin for use according to any of claims 1 to 6, wherein riboflavin is administered at a dosage from about 0.07 mg/kg to about 0.71 mg/kg, preferably from about 0.14 mg/kg to about 0.42 mg/kg, more preferably from about 0.14 mg/kg to about 0.28 mg/kg or from about 0.16 mg/kg to about 0.24 mg/kg.

**8.** Riboflavin for use according to any of claims 1 to 7, wherein oxaloacetate is administered at a dosage of below 4 mg/kg, preferably below 3 mg/kg, more preferably below 2.5 mg/kg.

**9.** Riboflavin for use according to any of claims 1 to 8, wherein said disease is acute ischemic stroke and the combination is administered within the first twelve hours of symptom onset, preferably within the first six hours, more preferably within the first three hours.

**10.** A composition comprising riboflavin and oxaloacetate.

**11.** A composition according to claim 10 wherein said composition is a pharmaceutical composition further comprising a pharmaceutically acceptable carrier, additive and/or excipient.

**12.** The composition according to claim 10 or the pharmaceutical composition according to claim 11 wherein said composition comprises riboflavin and oxaloacetate in the dosages defined in any of claims 7 or 8.

**13.** A kit of parts comprising at least two recipients wherein one of the recipients comprises a composition comprising riboflavin, and the other recipient a composition comprising oxaloacetate, together with instructions for use in the treatment of a disease involving the simultaneous or sequential administration of both active ingredients.

**14.** The pharmaceutical composition according to claim 11 or the kit of parts according to claim 12for use as defined in

any of claims 1 to 9.

**15.** Riboflavin or a pharmaceutical composition comprising thereof, for use in the therapeutic treatment of acute ischemic stroke by intravascular administration to a subject suffering or suspected to suffer from acute ischemic stroke within the first twelve hours of symptom onset, preferably within the first six hours of symptom onset, more preferably within the first three hours.

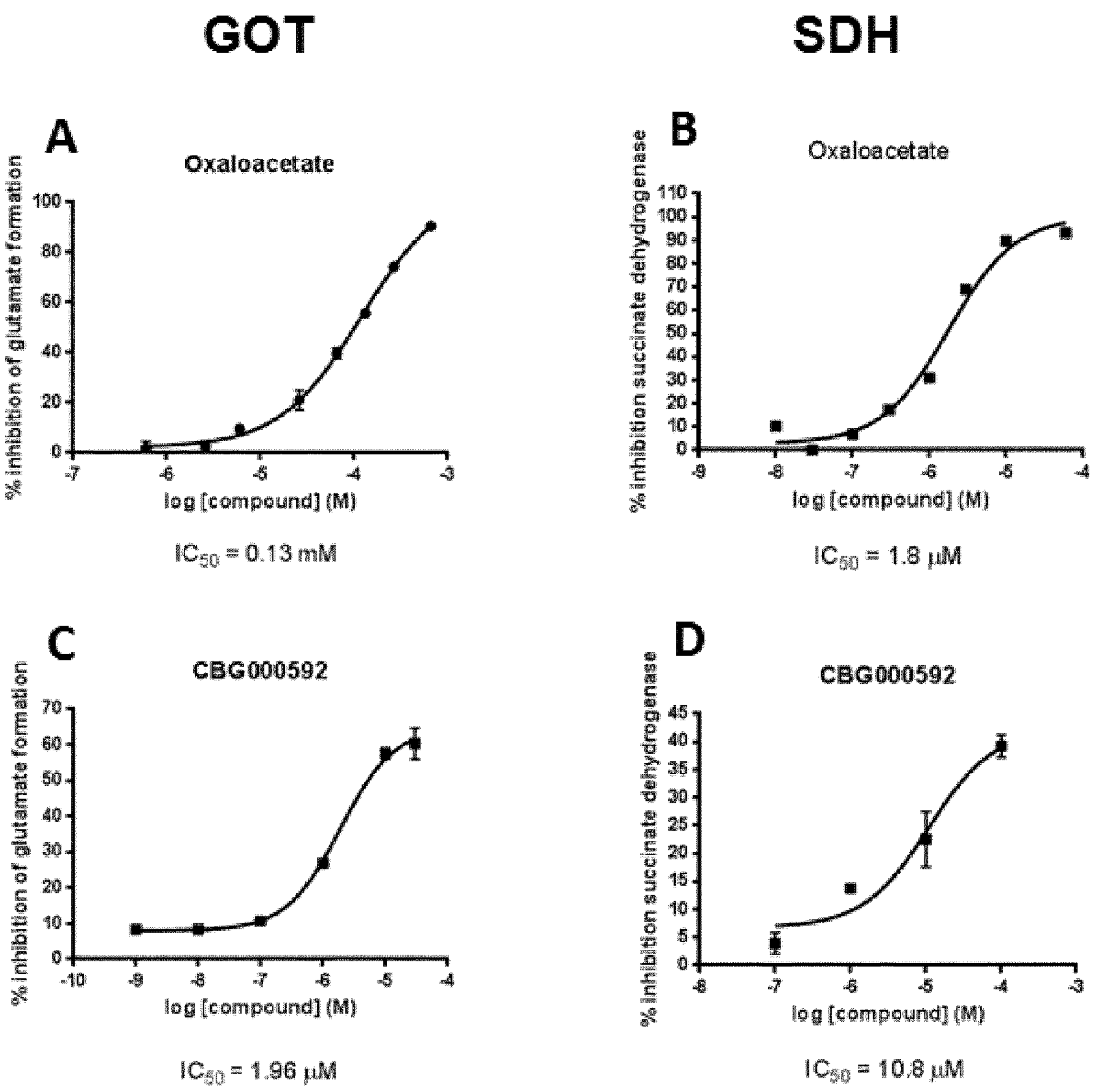
GOT
SDH
A
Oxaloacetate
% inhibition of glutamate formation
log [compound] (M)
$IC_{50}$ = 0.13 mM
B
Oxaloacetate
% inhibition succinate dehydrogenase
log [compound] (M)
$IC_{50}$ = 1.8 μM
C
CBG000592
% inhibition of glutamate formation
log [compound] (M)
$IC_{50}$ = 1.96 μM
D
CBG000592
% inhibition succinate dehydrogenase
log [compound] (M)
$IC_{50}$ = 10.8 μM

**FIG. 1**

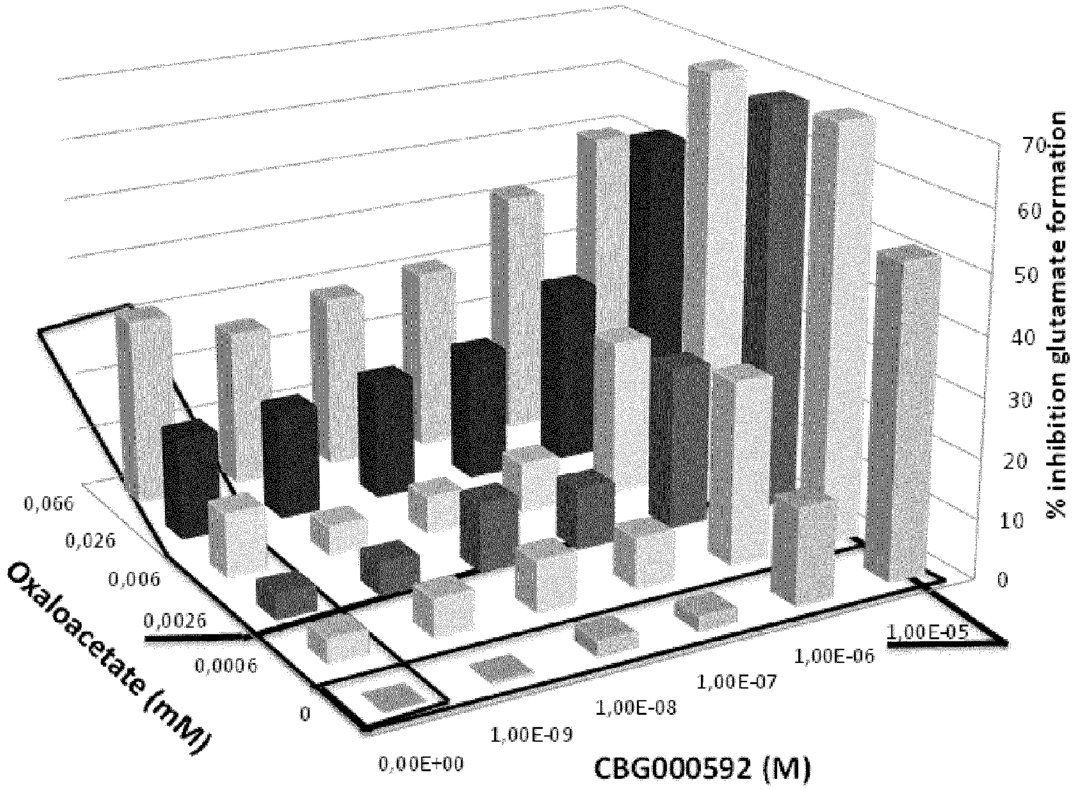
% inhibition glutamate formation
70
60
50
40
30
20
10
0
0,066
0,026
0,006
0,0026
0,0006
0
Oxaloacetate (mM)
0,00E+00
1,00E-09
1,00E-08
1,00E-07
1,00E-06
1,00E-05
CBG000592 (M)

FIG. 2

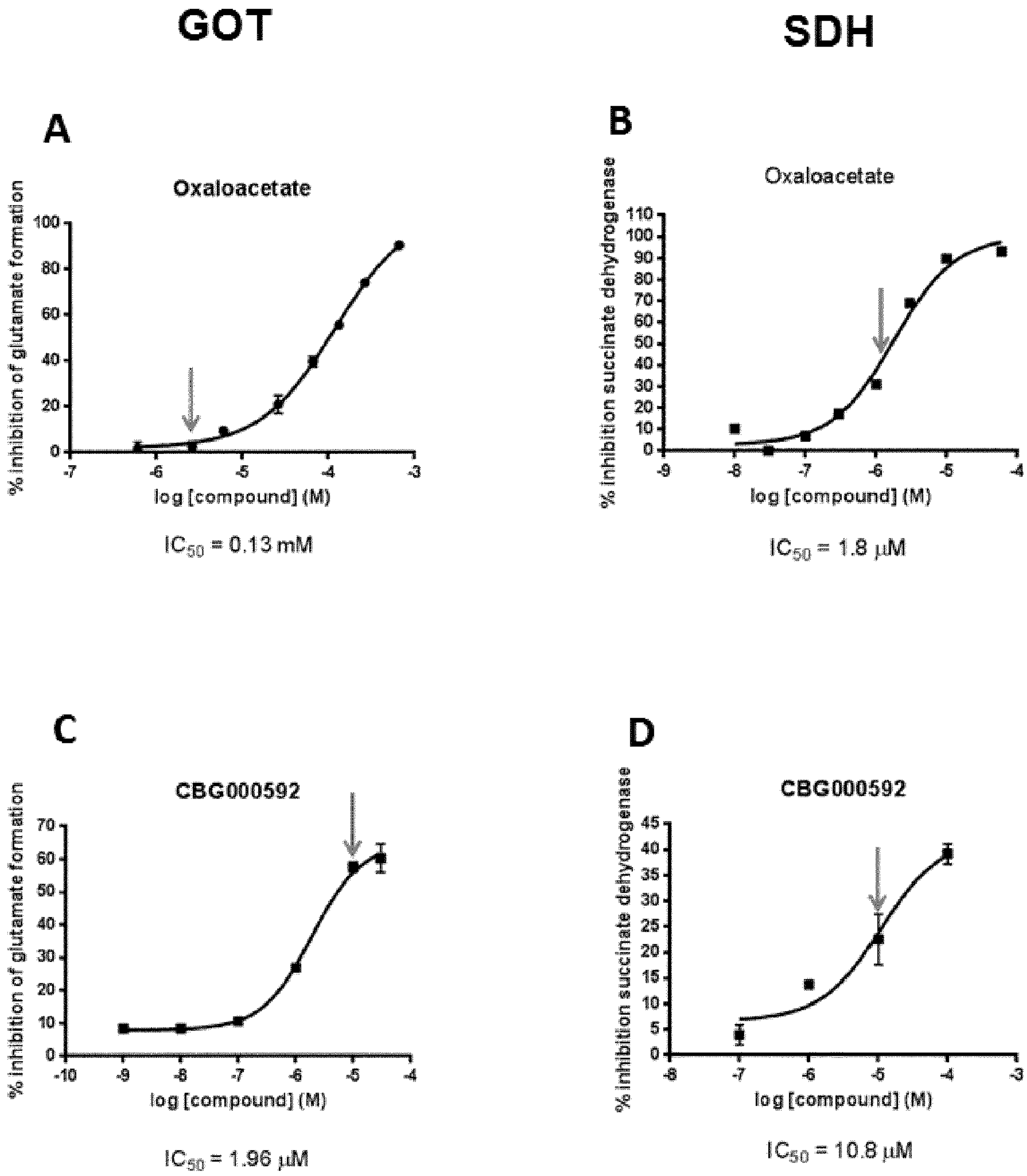
GOT
SDH
A
Oxaloacetate
% inhibition of glutamate formation
log [compound] (M)
IC50 = 0.13 mM
B
Oxaloacetate
% inhibition succinate dehydrogenase
log [compound] (M)
IC50 = 1.8 μM
C
CBG000592
% inhibition of glutamate formation
log [compound] (M)
IC50 = 1.96 μM
D
CBG000592
% inhibition succinate dehydrogenase
log [compound] (M)
IC50 = 10.8 μM

FIG. 3

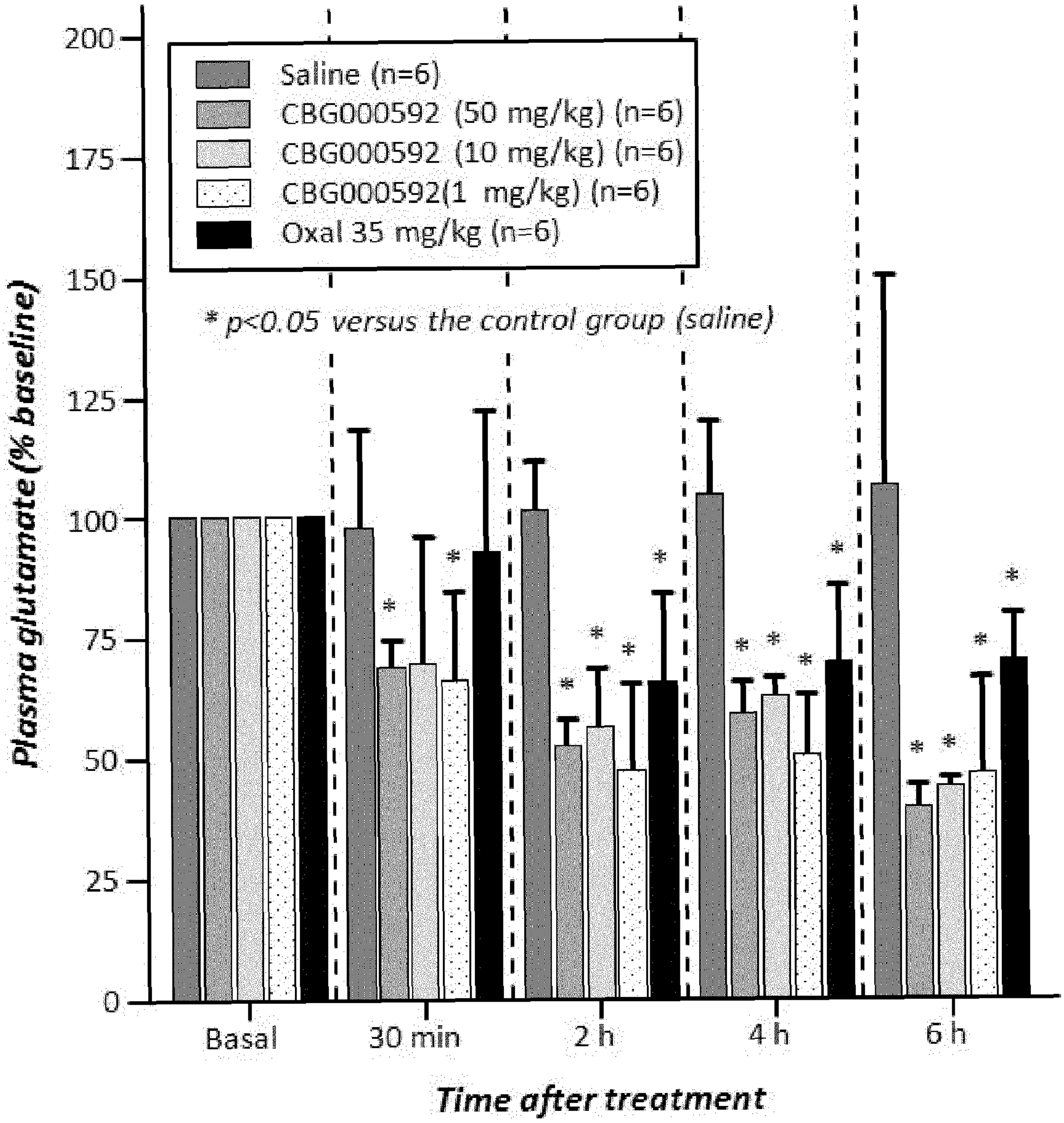
Saline (n=6)
CBG000592 (50 mg/kg) (n=6)
CBG000592 (10 mg/kg) (n=6)
CBG000592(1 mg/kg) (n=6)
Oxal 35 mg/kg (n=6)
* p<0.05 versus the control group (saline)
Plasma glutamate (% baseline)
0
25
50
75
100
125
150
175
200
Basal
30 min
2 h
4 h
6 h
Time after treatment

FIG. 4

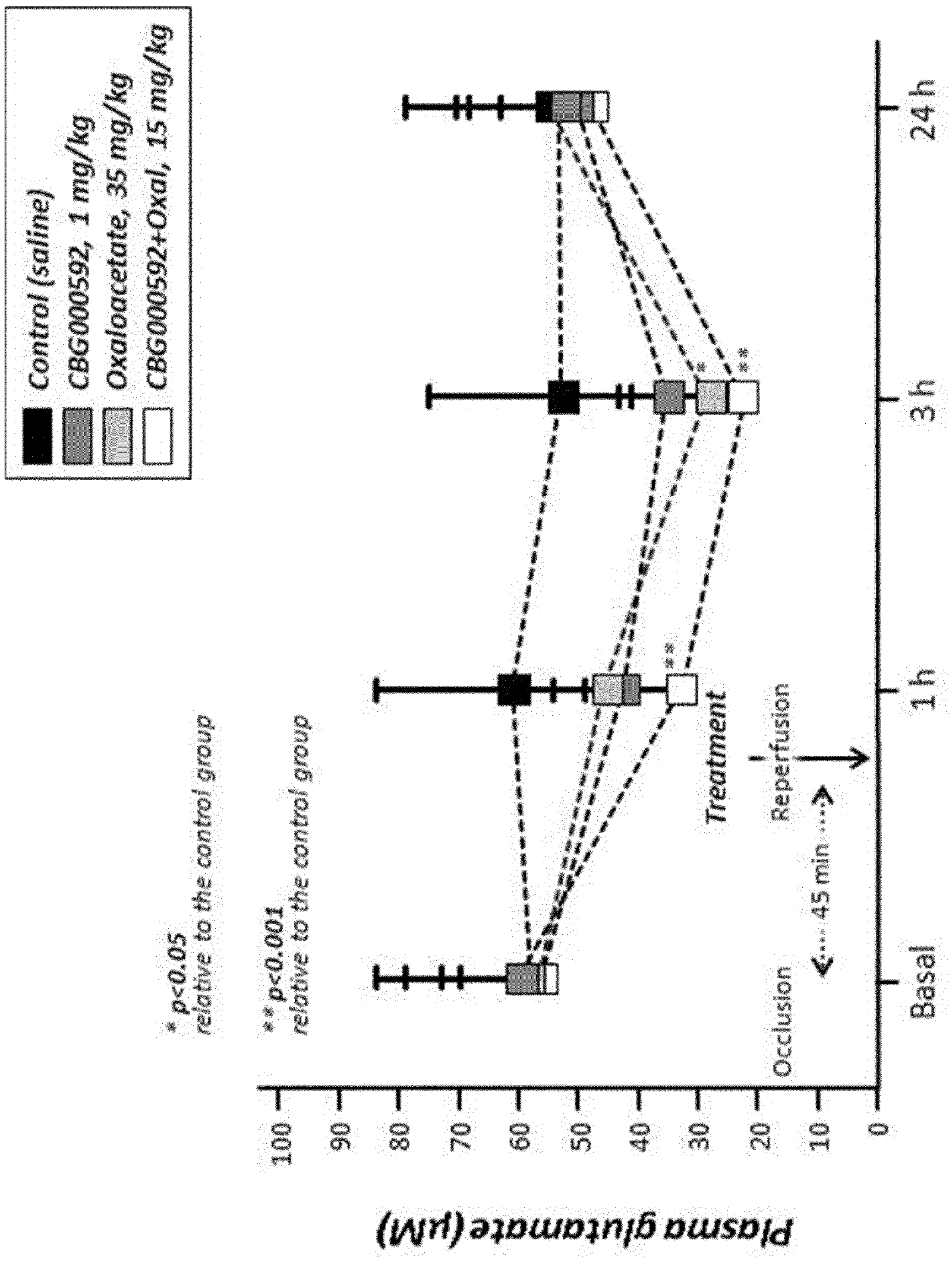
Control (saline)
CBG000592, 1 mg/kg
Oxaloacetate, 35 mg/kg
CBG000592+Oxal, 15 mg/kg
* p<0.05
relative to the control group
** p<0.001
relative to the control group
Plasma glutamate (µM)
100
90
80
70
60
50
40
30
20
10
0
Basal
1 h
3 h
24 h
Occlusion
45 min
Reperfusion
Treatment
*
**
**

FIG. 5

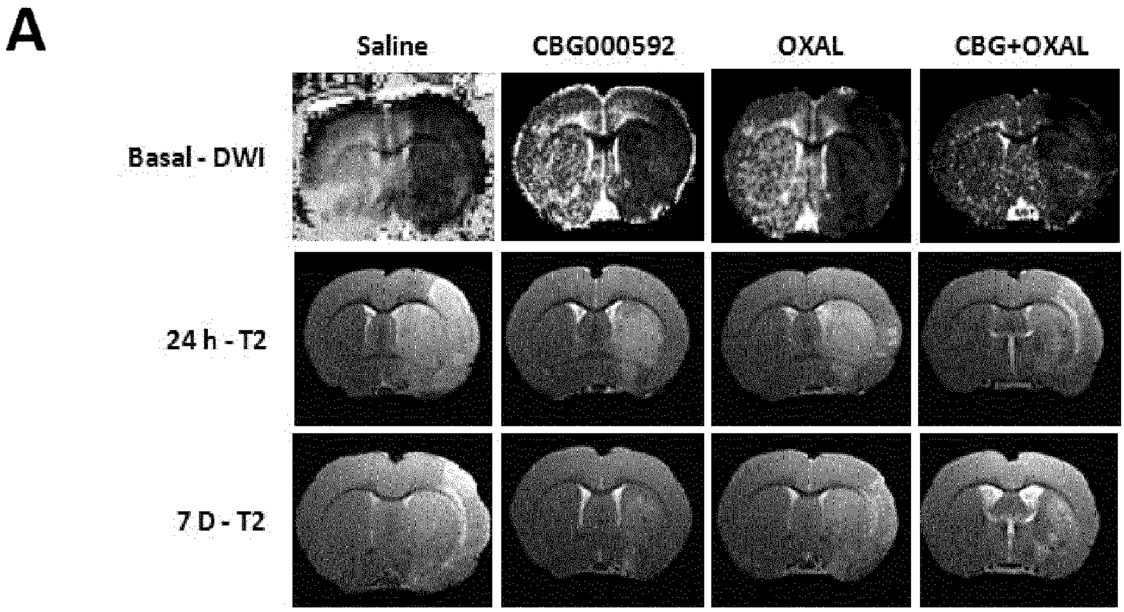


FIG.6

B

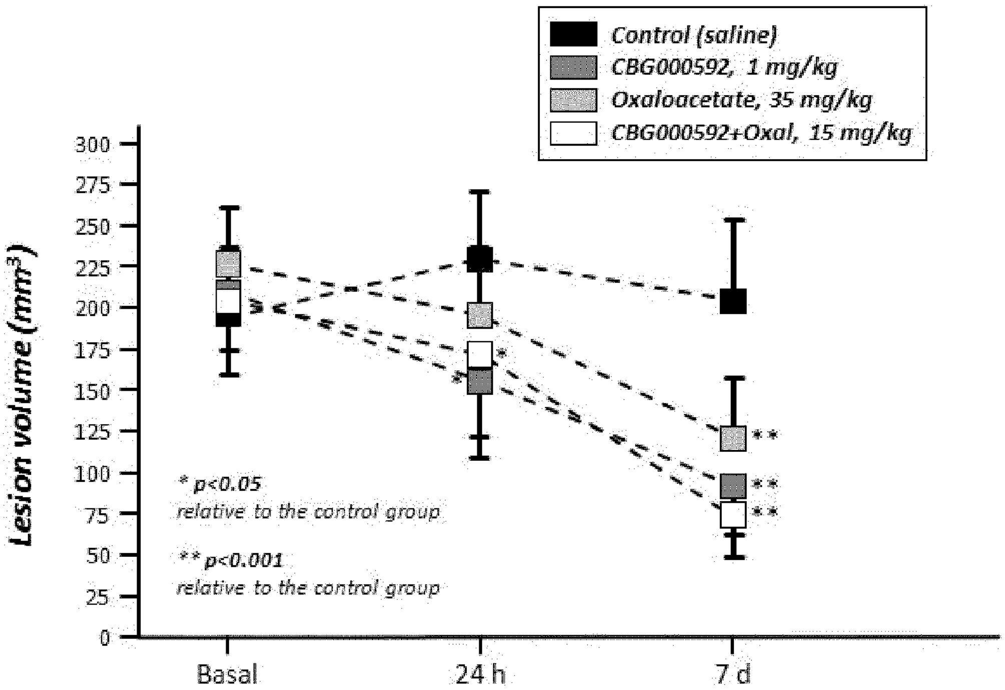
Control (saline)
CBG000592, 1 mg/kg
Oxaloacetate, 35 mg/kg
CBG000592+Oxal, 15 mg/kg
Lesion volume (mm³)
300
275
250
225
200
175
150
125
100
75
50
25
0
* p<0.05
relative to the control group
** p<0.001
relative to the control group
Basal
24 h
7 d

C

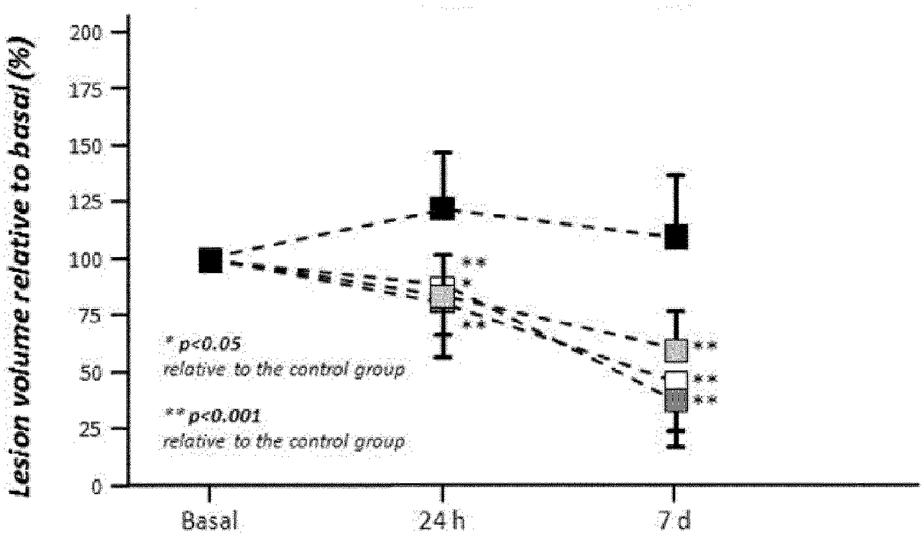
Lesion volume relative to basal (%)
200
175
150
125
100
75
50
25
0
* p<0.05
relative to the control group
** p<0.001
relative to the control group
Basal
24 h
7 d

FIG.6 (cont.)

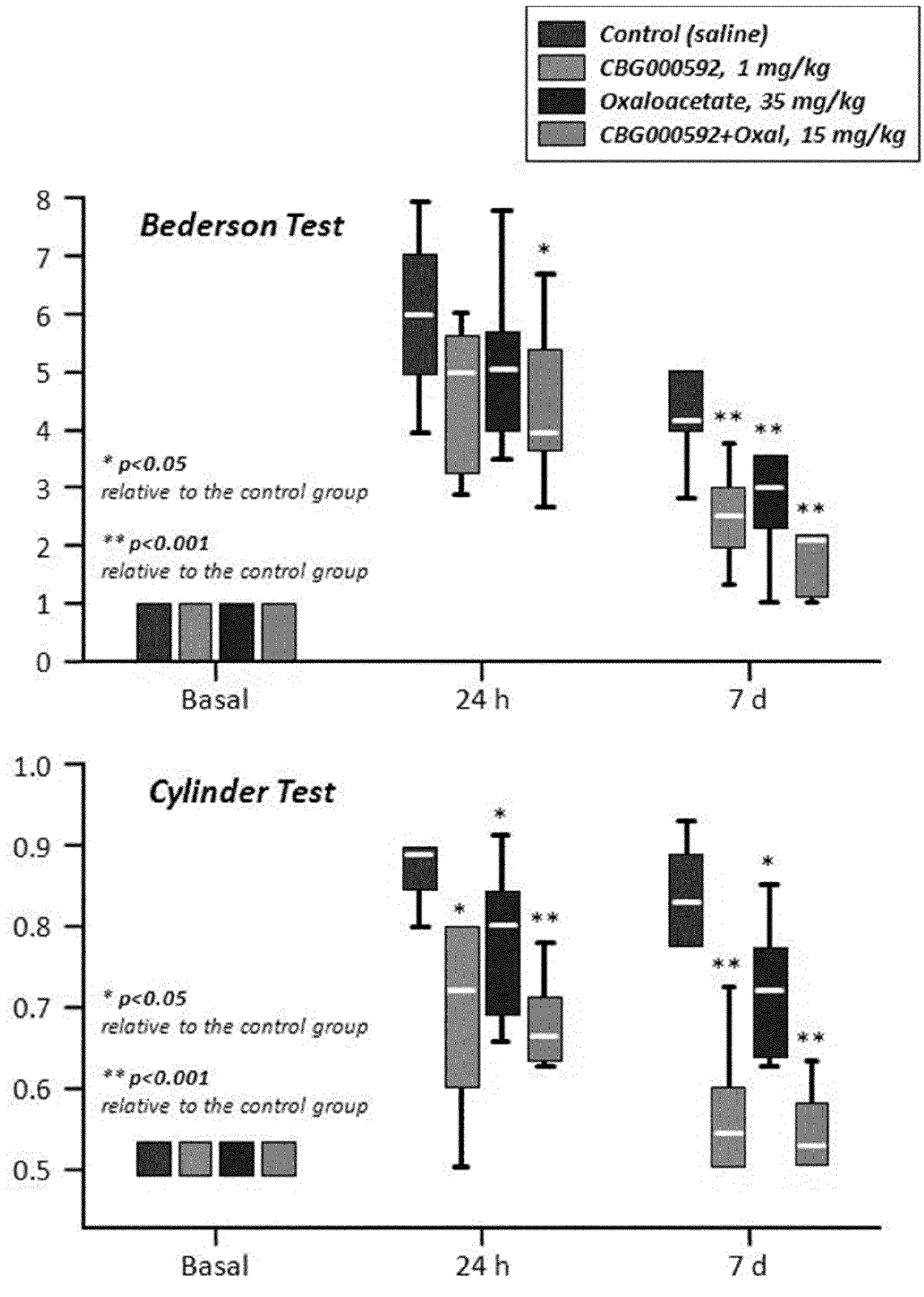
Control (saline)
CBG000592, 1 mg/kg
Oxaloacetate, 35 mg/kg
CBG000592+Oxal, 15 mg/kg
Bederson Test
* p<0.05
relative to the control group
** p<0.001
relative to the control group
0
1
2
3
4
5
6
7
8
Basal
24 h
7 d
Cylinder Test
* p<0.05
relative to the control group
** p<0.001
relative to the control group
0.5
0.6
0.7
0.8
0.9
1.0
Basal
24 h
7 d

FIG. 7

Europäisches Patentamt
European Patent Office
Office européen des brevets

# EUROPEAN SEARCH REPORT

Application Number
EP 15 38 2229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/066603 A2 (CENTOCOR INC [US]) 29 August 2002 (2002-08-29) * claim 4 * | 10-13 | INV. A61K31/19 A61K31/525 A61P9/10 A61P25/00 |
| X | WO 2011/148203 A1 (NOVAK MAJA [HR]) 1 December 2011 (2011-12-01) * example 1 * | 10-13 | |
| X | WO 93/10784 A1 (UNIV MICHIGAN [US]) 10 June 1993 (1993-06-10) | 15 | |
| Y | * page 3, lines 25-29 * * page 4, lines 22-27 * * page 10, lines 6-17 * | 1,2,4-9, 14 | |
| X | YING-XIN ZOU ET AL: "Importance of Riboflavin Kinase in the Pathogenesis of Stroke", CNS NEUROSCIENCE & THERAPEUTICS, vol. 18, no. 10, 24 August 2012 (2012-08-24), pages 834-840, XP055228404, GB ISSN: 1755-5930, DOI: 10.1111/j.1755-5949.2012.00379.x | 15 | |
| Y | * abstract * * figures 2,3 * | 1,2,4-9, 14 | |
| | -/-- | | |

| TECHNICAL FIELDS SEARCHED (IPC) |
|---|
| A61K |
| A61P |

The present search report has been drawn up for all claims

1

| Place of search | Date of completion of the search | Examiner |
|---|---|---|
| Munich | 16 November 2015 | Rodríguez-Palmero, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 38 2229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | FRANCISCO CAMPOS ET AL: "Oxaloacetate: A novel neuroprotective for acute ischemic stroke", INTERNATIONAL JOURNAL OF BIOCHEMISTRY AND CELL BIOLOGY, PERGAMON, GB, vol. 44, no. 2, 10 November 2011 (2011-11-10), pages 262-265, XP028435678, ISSN: 1357-2725, DOI: 10.1016/J.BIOCEL.2011.11.003 [retrieved on 2011-11-10] * abstract * * page 263, column 1, last paragraph - column 2, last paragraph * ----- | 1,2,4-9, 14 | |
| Y,D | TEICHBERG V I ET AL: "Homeostasis of glutamate in brain fluids: An accelerated brain-to-blood efflux of excess glutamate is produced by blood glutamate scavenging and offers protection from neuropathologies", NEUROSCIENCE, NEW YORK, NY, US, vol. 158, no. 1, 12 January 2009 (2009-01-12), pages 301-308, XP025924705, ISSN: 0306-4522, DOI: 10.1016/J.NEUROSCIENCE.2008.02.075 [retrieved on 2008-03-18] * abstract * * page 305, column 2, paragraph 3 - page 306, column 2, paragraph 2 * ----- | 1,2,4-9, 14 | |
| | -/-- | | TECHNICAL FIELDS SEARCHED (IPC) |

The present search report has been drawn up for all claims

1

| Place of search | Date of completion of the search | Examiner |
|---|---|---|
| Munich | 16 November 2015 | Rodríguez-Palmero, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 38 2229

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | JIA MING ET AL: "Taming glutamate excitotoxicity: strategic pathway modulation for neuroprotection", CNS DRUGS, ADIS INTERNATIONAL, AUCKLAND, NZ, vol. 29, no. 2, 30 January 2015 (2015-01-30), pages 153-162, XP009187162, ISSN: 1172-7047, DOI: 10.1007/S40263-015-0225-3 * abstract * * page 156, column 2, paragraph 2 - page 160, column 1, paragraph 2 * ----- | 1,2,4-9, 14 | |

TECHNICAL FIELDS SEARCHED (IPC)

The present search report has been drawn up for all claims

1

| Place of search | Date of completion of the search | Examiner |
|---|---|---|
| Munich | 16 November 2015 | Rodríguez-Palmero, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 15 38 2229

This annex lists the patent family members relating to the patent documents cited in the above-mentioned European search report. The members are as contained in the European Patent Office EDP file on
The European Patent Office is in no way liable for these particulars which are merely given for the purpose of information.

16-11-2015

| Patent document cited in search report | Publication date | Patent family member(s) | Publication date |
|---|---|---|---|
| WO 02066603 A2 | 29-08-2002 | AT 420945 T | 15-01-2009 |
| | | CA 2438148 A1 | 29-08-2002 |
| | | EP 1360314 A2 | 12-11-2003 |
| | | JP 2005505240 A | 24-02-2005 |
| | | JP 2008178419 A | 07-08-2008 |
| | | US 2003096402 A1 | 22-05-2003 |
| | | WO 02066603 A2 | 29-08-2002 |
| WO 2011148203 A1 | 01-12-2011 | EP 2575807 A1 | 10-04-2013 |
| | | HR P20140321 T1 | 23-05-2014 |
| | | WO 2011148203 A1 | 01-12-2011 |
| WO 9310784 A1 | 10-06-1993 | AU 3222893 A | 28-06-1993 |
| | | WO 9310784 A1 | 10-06-1993 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011148014 A


### Non-patent literature cited in the description


- **TOMSICK TA et al.** *Neurology,* 2010, vol. 74, 1069-1076
- **JIA M et al.** *CNS Drugs,* 2015, vol. 29, 153-162
- **LIPTON P. et al.** *Physiol Rev.,* 1999, vol. 79, 1431-1568
- **GINSBERG MD et al.** *Neuropharmacology,* 2008, vol. 55, 363-389
- **TEICHBERG et al.** *Neuroscience,* 2009, vol. 158, 301-308
- **CAMPOS et al.** *Int J Biochem Cell Biol.,* 2012, vol. 44, 262-265
- **CAMPOS F et al.** *J Cereb Blood Flow Metab.,* 2011, vol. 31, 1378-1386
- **LEIBOWITZ A. et al.** *Int J Mol Sei.,* 2012, vol. 13, 10041-10066
- **ARMSTRONG C ; STAPLES J.** *J Comp Physiol B.,* 2010, vol. 180, 775-783
- **KIRSCH et al.** *J Mol Biol,* 1984, vol. 174, 497-525
- **RUSTIN P et al.** *Biochem J.,* 1991, vol. 274, 249-255
- **KASNER SE.** *Lancet Neurol.,* 2006, vol. 7, 603-12
- **CASTILLO J et al.** *Lancet,* 1997, vol. 349, 79-83
- **CASTILLO J et al.** *Stroke,* 1996, vol. 27, 1060-1065
- **CASTILLO J et al.** *Cerebrovasc Dis.,* 1999, vol. 9, 22-27
- **CASTILLO J et al.** *Stroke,* 1995, vol. 26, 2035-2039
- *Am Fam Physician.,* 2015, vol. 91 (8), 528-36
- **ZEMPLENI et al.** *Am J Clin Nutr,* 1996, vol. 63, 54-66
- *report of the Expert Group on Vitamins and Minerals,* 2003, 68-73, http://cot.food.gov.uk/sites/default/files/vitmin2003.pdf
- Remington's Pharmaceutical Sciences. 2012
- **CAMPOS F. et al.** *J Cereb Blood Flow Metab.,* 2011, vol. 31, 1378-1386
- **PEREZ-MATO M et al.** *Cell Death Dis.,* 2014, vol. 5, e992
- **PÉREZ-MATO, M. et al.** *Cell Death Dis.,* 2014, vol. 5, e992
- **REAGAN-SHAW et al.** Dose translation from animal to human studies revisited. *The FASEB Journal,* 2007, vol. 22, 659-661
- **CAMPOS F. et al.** *, J Cereb Blood Flow Metab.,* 2011, vol. 31, 1378-1386
- **ALBERS GW et al.** *Stroke,* 1999, vol. 30, 2752-2758
- **PHILIP M et al.** *Stroke,* 2009, vol. 40, 577-581
- **RASBAND WS.** ImageJ. US National Institutes of Health, 1997
- **SCHALLERT T et al.** *Neuropharmacology,* 2000, vol. 39, 777-787
- **SCHAAR KL et al.** *Exp Transl Stroke Med.,* 2010, vol. 2, 13
- **STAPLES J.** *J Comp Physiol B.,* 2010, vol. 180, 775-783
- **CAMPOS F. et al.** *J Cereb Blood Flow Metab,* 2011, vol. 31, 1378-1386